**Europäisches Patentamt**

**European Patent Office**

**Office européen des brevets**

(11) Publication number: **0 335 134 B1**

# EUROPEAN PATENT SPECIFICATION

(45) Date of publication of patent specification: **01.03.95**

(51) Int. Cl.[6]: **C12P 21/08**, C12N 5/20, G01N 33/569, //C07K1/00, C07K7/00

(21) Application number: **89103859.8**

(22) Date of filing: **06.03.89**

(54) **Mouse monoclonal antibody to human immunodeficiency virus gp41 protein.**

(30) Priority: **30.03.88 US 176077**

(43) Date of publication of application:
**04.10.89 Bulletin 89/40**

(45) Publication of the grant of the patent:
**01.03.95 Bulletin 95/09**

(84) Designated Contracting States:
**AT BE CH DE ES FR GB IT LI NL**

(56) References cited:
**EP-A- 0 214 709**
**EP-A- 0 248 534**

**JOURNAL OF CLINICAL MICROBIOLOY, vol. 25, no. 5, May 1987, pages 845-848, American Society for Microbiology, Washington, US; L.H. GOSTING et al.:"Monoclonal antibodies to gp110 and gp41 of human immunodeficiency virus"**

(73) Proprietor: **ABBOTT LABORATORIES CHAD-0377, AP6D/2, One Abbott Park Road Abbott Park, Illinois 60064-3500 (US)**

(72) Inventor: **Hunt, Jeffrey C.**
**844 Paine Avenue**
**Lindenhurst**
**Illinois 60046 (US)**
Inventor: **Dawson, George J.**
**15670 Sprucewood Lane**
**Libertyville**
**Illinois 60048 (US)**
Inventor: **Sarin, Virender K.**
**516 Fairlawn Avenue**
**Libertyville**
**Illinois 60048 (US)**
Inventor: **Wray, Larry K.**
**1092 Princeton Avenue**
**Highland Park**
**Illinois 60035 (US)**

JOURNAL OF IMMUNOLOGY, vol. 139, no. 12, 15th December 1987, pages 4027-4033,American Association of Immunologists, Baltimore, US; B. BANAPOUR et al.:"Characterization and epitope mapping of a human monoclonal antibody reactivewith the envelope glycoprotein of human immunodeficiency virus"

NATURE, vol. 313, 7th February 1985, pages 450-458; M.A. MUESING et al.:"Nucleic acid structure and expression of the human AIDS/lymphadenopathy retrovirus"

Inventor: **Devare, Sushil G.**
**2492 Farnsworth Lane**
**Northbrook**
**Illinois 60062 (US)**
Inventor: **Webber, J. Scott**
**404 Keith Avenue**
**Waukegan**
**Illinois 60085 (US)**
Inventor: **Falk, Lawrence A.**
**448 N. County**
**Waukegan**
**Illinois 60085 (US)**

(74) Representative: **Modiano, Guido, Dr.-Ing. et al**
**Modiano & Associati S.r.l.**
**Via Meravigli, 16**
**I-20123 Milano (IT)**

## Description

BACKGROUND OF THE INVENTION

The present invention relates to the detection in body fluids such as blood or other blood products of exposure to the etiological agent of acquired immunodeficiency syndrome (AIDS). The etiological agent of AIDS is the human exogenous retrovirus termed Human Immunodeficiency Virus (HIV I), formerly named Human T-Cell Lymphotrophic Virus Type III (HTLV III), Lymphadenopathy Associated Virus (LAV), or AIDS Associated Retrovirus (ARV). In particular, a mouse monoclonal antibody, 5-21-3, and its native, conformation-dependent epitope within HIV I gp41 are provided which form the basis of an immunoassay used for the detection of exposure to HIV I. In another aspect, the invention relates to a monoclonal antibody for use as a probe against native antigens and synthetic peptides of HIV I.

Because AIDS is an infectious disease transmitted by parenteral exposure to contaminated blood or blood products (or intimate sexual contact), there is an established need to screen all blood and blood products prior to transfusion to detect and eliminate those derived from individuals exposed to HIV I. (See, for example Curran et al., Science (1985) 229:1352-1357; Council on Scientific Affairs, JAMA (1985) 254:1342-1345; Fauci et al., Ann. Int. Med. (1985) 102:800-813; Fauci et al., Int. Arch. Allergy Appl. Immun. (1985) 77:81-88. Diagnostic tests may determine if an individual is infected with HIV I by in vitro recovery of infectious virus from the individual. In addition, diagnostic tests may detect HIV I proteins or nucleic acid in body fluid or tissue, indicating possible HIV I infection. However, most diagnostic tests which are presently used for mass screening of blood and blood products are designed to detect antibody to HIV I, thus establishing prior exposure to HIV I.

The most widely used antibody test is the enzyme-linked immunoabsorbent assay (ELISA) employing inactivated whole virus cultured in a cell line capable of virus replication as the antigen reagent. While this method is quite sensitive, and there is a high likelihood that a truly infected person will be detected, there is also the possibility that persons never exposed to the virus may occasionally give positive results.

The detection of false positives in screening assays of blood products is one of the most pressing problems with the commonly used methods. Presently, specimen samples are screened by an ELISA method and if the result is positive, two more tests are run on the sample. If either is positive, the specimen is said to be repeatedly reactive. The Western Blot is the method commonly used to determine whether repeatedly reactive specimens contain antibodies to HIV. However, low value repeatedly reactive specimens often do not give a positive result on the Western Blot (Dassey et al.,JAMA,255, 743-745 (1986)). Nevertheless, the blood product must be disposed of because doubt exists regarding its infectivity (Chalmers et al., JAMA,256, 1778-1783 (1986)). These false positives are often due to nonspecific binding of immunoglobulins to cellular protein in the viral isolates.

Safety is another concern associated with the ELISA assay method because the procedures entail culturing live virus in vitro and subsequent isolation and deactivation to provide whole virus reagent.The infectious nature of the HIV virus makes manufacturing the virus potentially hazardous.

Scientists are currently developing second-generation tests in an effort to overcome the aforementioned problems associated with current assay methodologies. One approach has been the use of genetically engineered HIV antigens. Alternatively, tests may employ labeled antibodies directed toward the immunogenic epitopes of HIV I as competitive probes against antibodies in donated blood (or blood products). In addition, antibodies may be used as probes for detection of viral antigens. By identifying immunogenic HIV I proteins and their native, immunodominant regions or epitopes, and raising monoclonal antibodies specific to these epitopes, highly sensitive and specific diagnostic tests to determine exposure to HIV I can be produced. Additionally, the use of monoclonal antibodies as test reagents in diagnostic assays for AIDS avoids the hazard of working with the whole virus.

SUMMARY OF THE INVENTION

It has now been found that mouse monoclonal antibody 5-21-3, and its native conformation-dependent, non-linear epitope within HIV I gp41 form the basis of an immunoassay used for the detection of exposure to HIV I in body fluids. The amino acids of HIV I gp41 which form the contact residues with the antigen combining site of the monoclonal antibody are located between residues 131 and 154 (amino acids 649 thru 672 as numbered by Ratner et al., Nature (1985) 313:277-283). The monoclonal antibody identifies a native antigenic determinant which cannot be mimicked by a linear peptide comprising the region (131-154), but can be partially mimicked by peptides comprising this region which also contain flanking sequence of varying length at the 3′ and/or 5′ ends. The additional flanking sequence either 5′ (amino end) of amino acid

EP 0 335 134 B1

131 or 3'(carboxy end) of amino acid 154, is required to put the linear amino acid sequence (131-154) in the proper antigenic conformation.

Appropriate labeling (for example, isotope, enzyme or fluorescein) of the monoclonal antibody yields a highly specific reagent which can be employed in immunoassays designed to detect prior exposure to HIV. In presently preferred forms, serum, plasma, saliva, or other biological samples collected from the patient are analyzed by immunoassay techniques such as radioimmunoassays, fluorescent immunoassays, or enzyme-linked immunosorbent assays in either direct or competitive forms. In an especially preferred assay procedure of the invention the design of the immunoassay places the labeled monoclonal antibody in competition with antibody positive sera (containing anti-HIV I gp41 antibody) for binding to the native epitope recognized by the monoclonal antibody. The monoclonal antibody can also be used as a probe against native antigens and synthetic peptides of HIV. Monoclonal antibody mapping to this general region (131-154), has not been previously identified as clinically useful for detection or evaluation of prior exposure to HIV I.

## DESCRIPTION OF THE DRAWINGS

Fig. 1 is an autoradiograph showing the specificity of monoclonal antibody 5-21-3 for HIV I gp160/41 by immunoprecipitation of metabolically radiolabeled gp160.

Fig. 2 is a Western Blot showing specificity of monoclonal antibody 5-21-3 for HIV I gp41.

Fig. 3 is a photograph of microscope slides showing specificity of monoclonal antibody 5-21-3 for HIV I by immunocytochemical analysis.

Fig. 4 is an illustration of the location of the constructed synthetic peptides (P) on the HIV I ENV gene, which were employed to map and characterize the epitope recognized by monoclonal antibody 5-21-3.

Fig. 5 is an immuno-dot blot analysis of the mapping of the epitope recognized by monoclonal antibody 5-21-3.

Fig. 6 illustrates the epitope mapping of monoclonal antibody 5-21-3 using synthetic peptides in a binding inhibition assay. Selected concentrations of synthetic peptides P58-130 (▲), P67-154 (●), and P121- 154 (■) were tested for their relative ability to block binding of monoclonal antibody 5-21-3 to recombinant-derived p41.

## DETAILED DESCRIPTION

The present invention provides novel means for determining the presence of HIV infection in body fluids through the use of a monoclonal antibody characterized by its specificity for an epitope on HIV I gp41 formed by a first sequence of amino acids

```
Ile-His-Ser-Leu-Ile-Glu-Glu-Ser-Gln-Asn-Gln-Gln-Glu-Lys-Asn-Glu-Gln-Glu-L
eu-Leu-Glu-Leu-Asp-Lys
```

with at least one flanking second amino acid sequence of at least 5 amino acids in length either 3' to the carboxy terminus or 5' to the amino terminus of the first sequence, with the flanking sequence having an amino acid sequence corresponding to native HIV I gp41. In a preferred embodiment of the present invention, the monoclonal antibody ("5-21-3") is described, which is useful as a test reagent in diagnostic assays. In presently preferred forms, body fluid samples from patients are analyzed by immunoassay techniques such as radioimmunoassays, fluorescent immunoassays, or enzyme-linked immunosorbent assays in either direct or competitive formats.

In another of its aspects, the present invention provides novel hybridoma cell lines (exemplified by murine-derived cell line ATCC HB -9628) and novel monoclonal antibodies secreted thereby (exemplified by the above-noted 5-21-3 monoclonal antibody). Antibodies of the invention may be unlabeled or labeled with suitable detectable immunoassay markers such as radiochemicals, enzymes, fluorescent compounds or chemiluminescent compounds.

In an especially preferred embodiment of the invention labelled 5-21-3 monoclonal antibody is employed in an enzyme-immunoassay (EIA) as a competitive probe against anti-HIV I antibody in serum samples for binding to full length recombinant derived HIV I gp41 bound to a solid support. The labeled 5-21-3 monoclonal antibody can also be employed in the Abbott ENVACOR Assay as disclosed in U.S.Patent Serial No. 020,282.

4

In addition to the general competitive binding assay techniques described in detail herein, immunoassays within the scope of the present invention include any assay format which is dependent on the formation of an antigen/antibody complex. Such assay formats include, for example, agglutination assays and solid phase immunometric assays such as the forward sandwich, reverse sandwich and simultaneous sandwich assays. Any of the above assay formats can be utilized for the detection of antibodies to HIV I as well as the detection of the HIV I virus or portions thereof.

Biological samples which are easily tested by the methods of the present invention include human and animal body fluids such as whole blood, serum, plasma, urine, saliva, lymphocyte or cell culture preparations, and purified and partially purified immunoglobulins. Solid supports which can be used in the immunoassays of the invention include wells of reaction trays, test tubes, beads, strips, membranes, microparticles or other solid supports which are well known to those skilled in the art. Any label capable of producing a detectable signal can be used in the assays of the invention. Representative labels include enzymatic, radioisotopic, fluorescent and chemiluminescent labels. Further, hapten/labeled anti-hapten systems such as a biotin/labeled anti-biotin system may be utilized in the inventive assays. Alternatively, one may employ a labeled specific binding partner for the antibody which may be, for example, a labeled antibody specific for the antibody employed or labeled antigen that binds to the antibody. Where the monoclonal antibody is derived from a murine source, a labeled anti-mouse immunoglobulin specific for the antibody employed in the method may be used. Additionally, the antigen reagent may be composed of inactivated whole virus, or partially purified, native, synthetic, or recombinantly-produced HIV I protein.

In addition, the materials for use in the assays of the invention are ideally suited for preparation of a kit. Such a kit may comprise a carrier means being compartmentalized to receive in close confinement one or more container means such as vials, test tubes, and the like. Each of the container means comprises one of the separate elements to be used in the method.

The following illustrative examples relate to the development of monoclonal antibodies which specifically react with an antigenic determinant of gp41 of HIV I. More specifically, Examples 1 and 2 relate to procedures whereby hybridoma cell lines secreting monoclonal antibodies were generated. Example 3 relates to characterization of monoclonal antibody 5-21-3 produced by a selected hybridoma cell line. Example 4 relates to an amplification method for producing greater amounts of the 5-21-3 antibody. Example 5 relates to assays performed to determine the activity and specificity of the 5-21-3 monoclonal antibody. Example 6 relates to an enzyme-linked immunoassay (EIA) based on the 5-21-3 monoclonal antibody and use of this EIA in detection of HIV seropositive individuals. Example 7 relates to the mapping and characterization of the gp41 epitope recognized by monoclonal antibody 5-21-3. Cell line 5-21-3 was deposited at the American Type Culture Collection, Rockville, Maryland, on January 21, 1988, under deposit accession number HB-9628. The deposit was converted to a Budapest Treaty deposit on December 6, 1990.

Example 1

Immunization of Mice

In the procedure for production of hybridoma cell line 5-21-3, BZH mice (obtained from Chella David, Mayo Clinic, Department of Immunology) were immunized with partially purified, detergent disrupted HIV I (HTLV III prototype strain obtained from R.C. Gallo, National Institutes of Health). HIV I was partially purified from infected H9 cells by (a) membrane filtration separating HIV I from cells, followed by (b) concentration of cell culture fluid containing HIV I, followed by (c) collection of virus by ultracentrifugation, followed by (d) resuspension of the virus and collection by centrifugation onto a 20% sucrose pad, followed by (e) sucrose density gradient banding of HIV I at a density of approximately 1.16, and (f) ultracentrifugation of banded virus to collect and concentrate HIV I. HIV I was disrupted by addition of 0.5% Triton X-100, followed by vigorous sonication at 4°C.

On day 1, mice received 40 ug of disrupted HIV I with 10 ug of S.typhimurium extract (RIBI Immunochem. Research Inc.) in 0.2 ml of Freunds Complete Adjuvant (Difco Laboratories) given subcutaneously s.c.) and intraperitoneally (i.p.). The second immunization was performed 18 days later when mice received 50 ug of HIV I and 10 ug of S.typhimurium extract given s.c. and i.p. in Freunds Incomplete Adjuvant (Difco Laboratories). For the 3rd, 4th, and 5th immunizations, at 34, 47, and 68 days after the first immunization, respectively, mice were immunized i.p. and s.c. with approximately 50 ug of HIV I in RIBI Adjuvant System (RIBI Immunochem. Research Inc.) containing 6,6′-dimycolyltrehalose and monophosphoryl lipid A. Mice were bled on days 26, 42, and 56. The immune response of immunized mice was assessed by assaying their sera for anti-HIV I antibody by enzyme-linked immunoassay and western blot.

A Enzyme-linked Immunoassay (EIA)

Sera from naive or immunized mice were serially diluted into 20 mM potassium phosphate buffer, pH 7.4, containing 0.15 M NaCl 5 mM EDTA, 20% normal goat serum, 10% fetal calf serum, 10 mM EGTA, and 0.2% Tween 20. The diluted sera were reacted against polystyrene beads directly coated with partially purified HIV I, or, alternatively, with HIV I bound to the bead via human anti-HIV I IgG (purified from serum of an HIV I seropositive individual). After 2 hours at 40°C, the beads were washed, and goat anti-mouse IgG (H + L) Horseradish Peroxidase (HRPO)- conjugated antibody was added. The beads were incubated 2 hours at 40°C, washed, and transferred to reaction tubes for addition of o-phenylenediamine:2HCl (OPD) color reagent. The reaction was stopped after 30 min. by addition of 1N $H_2SO_4$, and the absorbance at 492 nm was determined. The absorbance was directly proportional to the amount of mouse antibody bound to the beads. Reactivity of mouse sera to HIV I antigens on either bead was confirmed by addition of a human HIV I seropositive serum (20-50 ul) as a competitor for binding to HIV I. These assays demonstrated the presence of antibody to HIV I in the sera of immunized mice.

B. Western Blot

Approximately 500 ug of partially purified HIV I were treated with sodium dodecyl sulfate (SDS) and 2-mercaptoethanol at 95°C, and electrophoresed in a 12% polyacrylamide-SDS gel (Laemmli et al., Nature - (1970) 227:680-685). Proteins were transferred overnight from the gel to nitrocellulose by electrophoresis at 100 mamp, or transferred in 1-2 hr at 1.0 amp, in standard transfer buffer composed of 25 mM Tris (Hydroxymethyl) Aminomethane, 192 mM glycine, and 2.0% methanol, pH 8.3. (Towbin et al., Proc. Natl. Acad. Sci. (1979) 76:4350-4354.) After transferring the viral proteins and blocking the nitrocellulose with 20% fetal calf serum diluted in 10 mM Tris buffer (pH 8.0) containing 0.15 M NaCl, the nitrocellulose was cut into strips (each strip containing approximately 15 ug of viral protein) which were used to determine the presence of anti-HIV I antibody in test sera (or other samples). Reaction mixtures consisted of a nitrocellulose strip incubated with an appropriate amount of test sample in 2.5 ml of buffer (20 mM Tris, 1 mM EDTA, 0.2 M NaCl, 0.3% Triton X-100, and 2 mg/ml bovine serum albumin, pH 7.5) for 1-2 hours at room temperature. The strips were washed with buffered detergent (10 mM phosphate buffered saline (PBS), pH 7.5, containing 0.1% SDS and 0.5% Triton X-100, alternated with PBS containing 0.5 M NaCl and 0.5% Triton X-100), followed by addition of goat anti-mouse IgG antibody conjugated to HRPO. The strips were incubated for 1-2 hours at room temperature, followed by washing with buffered detergent. Finally, antibody bound to viral protein was visualized by addition of freshly prepared HRP color reagent (Biorad) (120 mg dissolved in 40 ml ice-cold methanol, then diluted into 200 ml Tris buffered saline, pH 7.8, containing 120 ul of 30% hydrogen peroxide). This assay demonstrated the presence of antibody to specific HIV I proteins.

Example 2

Cell Fusion

Upon demonstration of specific anti-HIV I antibody present at reasonable titers in sera of immunized mice, the mice were allowed to rest for at least 3 weeks prior to a pre-fusion boost of antigen. The pre-fusion antigen boost was performed by intravenous (tail vein) injection of approximately 40 ug of disrupted HIV I. Three days later the mice were sacrificed, and their spleens, containing anti-HIV I antibody producing cells, were disrupted to single cells. The single cell suspensions were treated with 0.83% $NH_4Cl$ to remove red blood cells, and then mixed with SP2/0 cells at a 10:1 (SP2/0:spleen cells) ratio. The mixed cells were centrifuged, washed once with serum-free medium, then centrifuged again. The fusogen, polyethylene glycol (PEG), was used to form hybrids of the immune donor spleen cells with the myeloma cell line SP2/0 (HPRT neg.) [G. Kohler and C. Milstein, Nature (1975) 256:494, and reviewed in Monoclonal Hybridoma Antibodies: Techniques and Applications, ed. J.G.R. Hurrell (CRC Press, Inc., 1982)]. Briefly, fusion of the spleen and SP2/0 cells was accomplished by exposing the pellet to 40% PEG (ATCC, MW 1300-1600) in serum-free Iscoe's Modified Dulbecco's Medium (IMDM) for two minutes. The PEG and cell suspension was diluted slowly by the addition of 20 ml of serum-free IMDM over a period of five minutes, followed by collection of the cells by centrifugation. The supernatant was decanted and replaced with 30 ml IMDM containing 20% fetal bovine serum (Hyclone) with HAT (hypozanthine, aminopterin, and thymidine) to select for hybridomas. Spleen cells from one nonimmune Balb/c mouse also were added as a feeder layer. The cells were plated at 0.1 ml/well in three 96 well tissue culture plates. Three days later an additional 0.1 ml of HAT media was added to each well. At weekly intervals thereafter, one-half the media was replaced with IMDM containing 20% fetal bovine serum (Hyclone) with HT (hypozanthine and thymidine), and hybrids were allowed to grow for an additional 7-14 days.

Some of the hybrids were composed of spleen cells making antibody to HIV I fused with SP2/0 cells. Briefly, the fusogen promotes fusion of spleen cell and SP2/0 cell membranes, forming a heterokaryon containing nuclei of both cells. Eventually, the dissimilar nuclei fuse producing a single nucleus capable of synchronous mitosis. As the fused cells divide, the hybrid stabilizes by losing chromosomes of each nuclei. The fused cells are plated into multiple 96 well plates at $10^5$ to $10^6$ cells per well. Hybrid cells formed from SP2/0:spleen cell fusions are selectively propagated by culturing in HAT medium. All unfused SP2/0 or SP2/0:SP2/0 fused cells are prevented from growing by aminopterin, and unfused spleen cells or spleen: spleen fused cells die off in culture. Only SP2/0:spleen hybrids will grow in the HAT selection medium.

Example 3

Screening, Cloning, and Characterization of Monoclonal Antibody

After 10-14 days, culture fluids from the wells containing cell growth (hybridomas) were screened for antibody to HIV I using procedures described in Example 1 (EIA and Western Blot). In addition, an EIA using gp41 produced in E .coli by recombinant DNA methods (rp41) was employed for screening. Briefly, polystyrene beads coated with human IgG (purified from an anti-gp41 seropositive individual) were reacted with crude extracts of E. coli previously induced for production of the HIV I gp41 cloned gene (BglII to KpnI restriction fragment, containing all the amino acids of gp41) in a lac z expression vector (Wood et al., Cold Spring Harbor Symposium (1985), p. 72: Wood et al., J. Cell. Biochem. Suppl. (1985) 9A:24). The resultant beads, bearing rp41 bound via human IgG, were reacted with hybridoma culture fluid for 2 hr at 40°C, washed, and then incubated with HRPO-labeled goat anti-mouse IgG antibody for 1 hr at 40°C. After washing again, beads were transferred to reaction tubes for addition of OPD substrate as described in Example 1.

From a single fusion (fusion #5), 16 wells were identified which contained cell growth and colony formation. Culture fluid from each well was screened by EIA and Western Blot. Of the 16 wells 7 were identified as possibly containing antibody to HIV I. Cells from each of these 7 were expanded in multiple 24 well plates, and culture media was again assayed for presence of anti-HIV I antibody. All 7 were positive for anti-HIV I antibody, and each hybrid was further expanded in T25 flasks for cloning by limiting dilution. Each expanded hybrid was plated into 96 well microtiter plates at dilutions of $10^5$ to $10^6$ and allowed to grow for 10-21 days. Among the clones identified as producing anti-gp41 antibody was clone #3 from hybrid #21 of fusion #5, which was named accordingly, 5-21-3. The clone was obtained by limiting dilution using the guidelines outlined by J.W. Goding in Monoclonal Antibodies: Principles and Practice (Academic Press, N.Y., 1983). The isotype of monoclonal antibody 5-21-3 was determined to be IgG1 The EIA isotyping procedure employed a microtiter plate coated with goat anti-mouse IgG immunoglobulin, which was incubated with culture fluid of the clone to capture the secreted mouse antibody. After 2 hours, the pate was washed, then rabbit anti-mouse isotype was applied for an additional 2 hr. The plate was washed again, and HRPO-conjugated goat anti-rabbit IgG was applied for 1 hr. The excess conjugate was removed by washing, then OPD substrate was added. The amount of rabbit anti-mouse isotype bound to the mouse immunoglobulin was proportional to the absorbance measured at 492 nm. Further characterization was performed with 5-21-3 antibody from mouse ascites.

Example 4

Amplification of Antibody Yields by Ascites Method

In order to obtain greater amounts of 5-21-3 monoclonal antibody, ten to twenty million cloned 5-21-3 cells were innoculated into a Balb/c mouse previously treated intraperitoneally with 0.5 ml pristane (2,6,10,14-tetramethylpentadecane)[method outlined in Hurrell et al., supra]. Pristane treatment enhances growth of mouse myeloma hybrids within the peritoneum of the mouse, and the ascites fluids which form are rich in the monoclonal antibody secreted by the hybrid cells. After formation of monoclonal antibody-enriched ascites, the mice were sacrificed, and the ascites withdrawn from the peritoneum was clarified by centrifugation and stored at -20°C. The monoclonal antibody in the ascites was titered by EIA (see Example 5). Other characterization procedures (described herein) were performed with the clarified ascites fluid or purified antibodies from the ascites, using several purification procedures known in the art, including Protein A-Sepharose (Hurrell et al., supra).

Example 5

Activity and Specificity

Assays were performed to determine the titer of monoclonal antibody in mouse ascites and to assess the specificity of the monoclonal antibody.

A. EIA

Assays described in Examples 1 and 3 were used to determine the titer of monoclonal antibody in ascites fluid. Briefly, clarified ascites fluid was reacted against polystyrene beads coated directly with partially purified HIV I, or, against beads bearing HIV I proteins bound via human IgG . Using these assays, the titers of antibody in ascites fluid were relatively low (1:8000 to 1:16,000). However, the activity was determined to be against HIV I protein because serum from a seropositive AIDS patient was a strong competitive inhibitor of the monoclonal antibody (Table 1).

In addition, a second assay was employed which utilized rp41 produced in E. coli by recombinant DNA methods (described in Example 3), and IgG from a goat immunized with HIV I. Goat anti-HIV I IgG bound to polystyrene beads was used to capture the rp41 (see Table 1, footnote d). The monoclonal antibody-enriched ascites exhibited high titers (>1 : 128,000) against the rp41 antigen. These results indicated that the monoclonal antibody was directed against an epitope present on HIV I gp41.

Table 1

| Activity of Monoclonal Antibody 5-21-3 Enriched Mouse Ascites Against Disrupted HIV I and rp41. | | | | |
|---|---|---|---|---|
| Ascites Dilution | ABSORBANCE(492nm) | | | |
| | HIV coated bead[a] | AB + HIB coated bead[b] | Competition w/AIDS serum[c] | AB + rp41 bead[d] |
| 1:2,000 | 0.214 | 0.291 | 0.114 | 1.149 |
| 1:4,000 | 0.222 | 0.247 | 0.074 | 1.042 |
| 1:8,000 | 0.171 | 0.165 | 0.059 | 1.062 |
| 1:16,000 | 0.130 | 0.160 | 0.039 | 0.825 |
| 1:32,000 | 0.099 | 0.099 | 0.032 | 0.687 |
| 1:64,000 | 0.054 | 0.067 | ND[e] | 0.678 |
| 1:128,000 | 0.023 | 0.062 | ND[e] | 0.434 |

[a]Partially purified, detergent disrupted HIV I was coated directly onto a polystyrene bead. The endpoint titer of 5-21-3 was considered to be 1:8,000 to 1:16,000.
[b]Anti-HIV I IgG purified from the serum of an AIDS patient was coated onto a polystyrene bead and used to capture HIV I proteins, which in turn served as the antigenic targets in the assay. The endpoint titer of 5-21-3 against these beads was considered to be 1:8,000 to 1:16,000.
[c]Serum from an AIDS patient (anti-HIV I) was used to block reactivity of 5-21-3 ascites against the AB + HIV bead, thus confirming the monoclonal antibody was seeing the same antigens as HIV I immune human serum.
[d]IgG purified from the serum of a goat immunized with HIV I, was coated onto a polystyrene bead and used to capture rp41 produced in E. coli by recombinant DNA methods. The endpoint titer of 5-21-3 enriched ascites was considered to be greater than 1:128,000 against the rp41 coated beads.
[e]ND, not determined.

B. Radioimmunoprecipitation (RIP)

Specificity of 5-21-3 antibody was confirmed by immunoprecipitation of[35S]methionine/[35S]-cysteinebiosynthetically-labeled viral glycoprotein gp160 (envelope precursor) from HIV I infected H9 cell lysates. Immunoprecipitation assays for viral proteins have been described previously (Devare et al., Proc. Natl. Acad. Sci., U.S.A. (1986) 83: 5718-5722). Cell lines employed for these studies were uninfected H9 cells, HIV I infected H9 cells, or a cloned cell line, S4C8, which was derived from HIV I infected H9 cells, but which produces primarily gp160, gp120, and gp41, and little or no gag gene products. Cells were harvested from culture, washed once with RPMI 1640 deficient in methionine and cysteine (Gibco Laboratories), then suspended at 1-2.5 x $10^6$ cells/ml in the same medium. Washed cells were incubated for 30- 45 min. at 37°C in 6% $CO_2$, followed by addition of 50-100 uCi each of [$^{35}$S]methionine and [$^{35}$S]-cysteine (Amersham) to the medium. Cells were radiolabeled at 37°C for 4-8 hr, harvested by centrifugation, and lysed in PBS, pH 7.4, containing 1 mM PMSF, aprotinin (100 Kallikrein inactivation units per ml of buffer), 1.0% Triton X-100, 0.1% SDS, and 0.5% sodium deoxycholate (all reagents from Sigma). The lysate was clarified by centrifugation at 100,000 x g for 40 min. and stored at - 70°C.

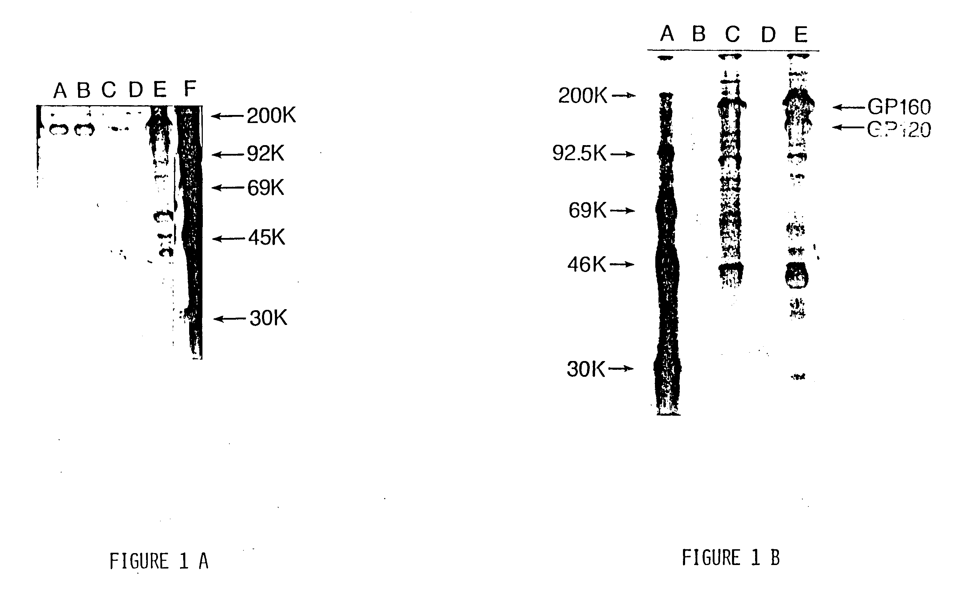

Immunoprecipitation was performed by incubating 100 ul aliquots of cell lysates with 5-20 ug of purified monoclonal antibody (1mg/ml), 3-5 ul of monoclonal antibody-enriched ascites, 3-10 ul of serum, or 100 ul tissue culture fluid, for 30-60 min. at 4°C. Antibody-antigen complexes were recovered by addition of 200 ul of pre-swollen Protein A-Sepharose (Pharmacia) previously washed in lysis buffer containing 1mg/ml bovine serum albumin (IgG binding capacity of 50-200 ug/200 ul Protein A). The reaction mixture was shaken vigorously at 4°C for 1 hr, followed by 3 washes of the Protein A-Sepharose using lysis buffer. Protein A- Sepharose was then collected by centrifugation, and immune complexes were dissociated by heating at 95°C in SDS gel sample buffer containing 2-mercaptoethanol (Laemmli et al., supra). The sample was subjected to SDS-10% polyacrylamide gel electrophoresis. The gel was incubated for 30 min. in Enhance (Dupont), dried, and exposed to x-ray film for autoradiography of the immunoprecipitated, radiolabeled proteins. The results are illustrated in Figure 1 where, in Panel A, cell lysates from labeled HIV I infected H9 cells were mixed with 3 ul of 5-21-3 enriched ascites (A), 3 ul of a different (not 5-21-3) anti-gp160/41 monoclonal antibody enriched ascites (B), 3 ul of normal mouse serum C), 3 ul of a miscellaneous mouse ascites (D), or 3 ul of a HIV I immune human serum (E). Molecular weight standards are in (F). In Panel B, cell lysates from labeled S4C8 cells (C,E) or uninfected H9 cells (B,D) were mixed with 100 ul of tissue culture fluid containing 5-21-3 (B,C) or 5 ul of HIV I immune human serum (D,E). Molecular weight standards are in (A). The results indicated that only gp160, and not gp120, was precipitated from HIV I infected cell lysates by the monoclonal antibody, and that there was no detectable cross reaction with any other radiolabeled viral or cellular protein. Thus, the monoclonal antibody 5-21-3 specifically bound HIV I gp160/41.

C. Western Blot

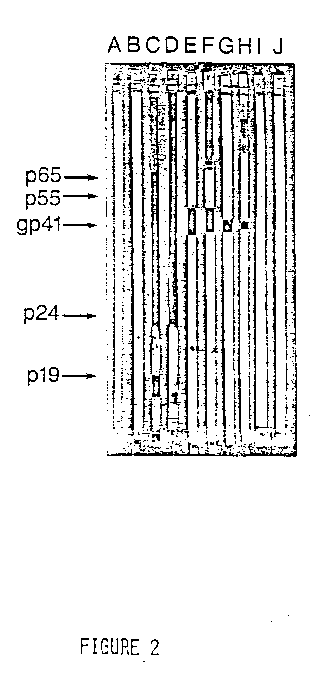

Western blot analysis (as described in Example 1) of purified monoclonal antibody 5-21-3 was performed to further establish specificity. The results are illustrated in Figure 2 where approximately 48 ug of partially purified HIV I was electrophoretically transferred onto each nitrocellulose strip after electrophoresis in a 12% polyacrylamide gel under reducing (A,C,E,G,I) or non-reducing (B,D,F,H,J) conditions. The strips were incubated with 1 .0% normal human plasma (A,B), 1.0% HIV I immune human serum (C,D), 15 ug/ml purified anti-HIV I gp41 monoclonal antibody 10-15-63 (E,F), 15 ug/ml monoclonal antibody 5-21-3 (G,H),or 15 ug/ml normal mouse IgG (I,J). The results indicated that 5-21-3 was specific for a single diffuse band of $M_r$ 41K when reacted against nitrocellulose strips bearing HIV I proteins. No other non-specific reactions were demonstrated. These data were consistent with the previously demonstrated specificity of the antibody toward gp160/41.

D. Immunocytochemical Assay (ICA)

Monoclonal antibody 5-21-3 was employed in an immunocytochemical assay against uninfected and HIV I infected H9 cells in order to further demonstrate its specificity for HIV I. Briefly, uninfected or HIV I infected H9 cells were fixed to microscope slides using 100% acetone for 10 min., then air dried, and stored at -20°C. For staining, the cells were rehydrated in PBS for 10 min., followed by addition of 100 ul of 2% normal goat serum in PBS containing 100 ug/ml normal human IgG for 10 min. Excess reagent was removed, and 100 ul of 1 ug/ml 5-21-3 IgG was added for 30 min. to the slides maintained in a humidified chamber. The slides were washed in PBS, followed by addition of 100 ul of the second antibody, goat anti mouse IgG (Pel Freez) at a 1:400 dilution, for 30 min. The slides were washed with PBS, followed by addition of 100 ul of peroxidase anti-peroxidase complex (Sternberger and Meyer, Clonal PAP) diluted 1:300 in PBS containing 2% normal goat serum and 100 ug/ml normal human IgG. The slides were incubated (with the Clonal PAP) in a humidified chamber. After washing in PBS, color development was initiated by the addition of 100 ul of the chromagen (0.63 mg/ml tetra hydrochloride diamino-benzidine in 0.06% hydrogen peroxide in PBS). Color development was stopped after 6 min. by washing the slides in tap water. The slides were dehydrated and mounted for examination.

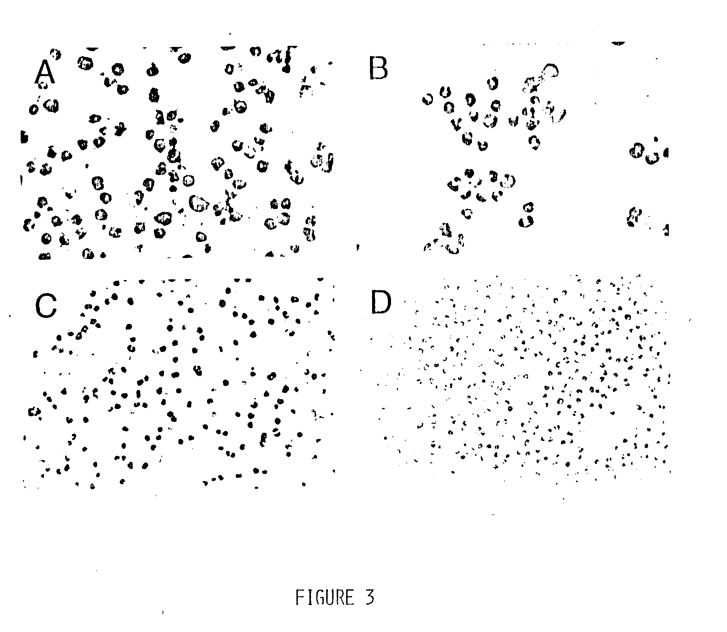

Results of the immunocytochemical analysis are illustrated in Figure 3 where the monoclonal antibody 5-21-3 (5 ug/ml) was reacted against (A) HIV I infected H9 cells (400x) and (B) uninfected H9 cells (400x). Normal mouse IgG (5 ug/ml) also was reacted against (C) HIV I infected H9 cells (250x) and (D) uninfected H9 cells (250x). Results indicated that 5-21-3 did not cross react with any normal cellular constituent. Specific staining was not detected when normal, uninfected H9 cells were employed as the target for 5-21-3 (panel B). In sharp contrast, a strong, characteristic pattern of staining was apparent when HIV I infected H9 cells were employed as the target (panel A). A characteristic "prominant body" was observed in the HIV I infected cells when the primary antibody was 5-21-3. This staining pattern was absent when normal mouse IgG was substituted for 5-21-3 IgG (panel C). In addition, the "prominant body" was absent when anti-HIV I p24 or anti-HIV I p17 monoclonal antibodies were used as the primary antibody (not shown), although they also exhibited characteristic staining patterns only against infected cells.

## Example 6

Clinical Utility of a Competitive EIA Employing Monoclonal Antibody 5-21-3 for Identification of HIV I Seropositive Individuals

### A. Competitive EIA

Monoclonal antibody 5-21-3 was purified from mouse ascites using Protein A-Sepharose, and coupled to horseradish peroxidase (HRPO). Briefly, the HRPO was activated by addition of 1.6 ml of 5 mg/ml HRPO (Sigma) to 1.6 ml of $NaIO_4$ at 17 mg/ml. The mixture was incubated 30 min. at 30°C in the dark, followed by separation of activated HRPO from $NaIO_4$ by G25 Sephadex gel filtration. Fractions containing activated HRPO were pooled and protein concentration was adjusted to approximately 0.6 mg/ml. One ml of 1 mg/ml purified 5-21-3 IgG, previously dialyzed into 50 mM sodium carbonate buffer, pH 9.5, was added to 1.1 ml of activated HRPO at 30°C and incubated 2 hr in the dark, followed by a 30 min. incubation at 4°C. The theoretical coupling ratio of HRPO:IgG in the reaction mixture was 4:1. Freshly prepared $Na_2BH_4$ (0.08 ml of 5 mg/ml solution in cold 10 mM $Na_2CO_3$, pH 9.5) was added to the mixture and left overnight at 4°C, followed by addition of 0.08 ml acetone which was allowed to react for 30 min. at 30°C. Finally, 7.7 ml of 1.43% bovine serum albumin in PBS (pH 7.2) were added to yield a final IgG concentration of 100 ug/ml; aliquots were stored at -70°C.

The conjugated monoclonal antibody ([HRPO]5-21-3) was employed in an EIA as a competitive probe (Monoclonal Assay) against anti-HIV I antibody in serum samples, for binding to full-length recombinant-derived HIV I rp41. Polystyrene beads were coated with human IgG (purified from sera of an HIV I seropositive individual having strong anti-gp41 activity), and then incubated with clarified E. coli extracts, previously induced for production of full-length rp41 (as described in Example 3). The beads bearing rp41 were used as serological targets for competition between [HRPO]5-21-3 and anti-HIV I seropositive samples. Beads were added to the wells of reaction trays (Abbott Laboratories) containing 50 ul of the test sera and 200 ul of [HRPO]5-21-3 diluted in PBS (pH 7.4) containing 10% normal human plasma, 10% fetal calf serum, 20% normal goat serum, 10% Triton X-100, and 4.77 g/L of HEPES buffer. This reaction mixture was incubated for 4 hr at 40°C, followed by vigorous washing, transfer of the bead to a reaction tube, and addition of 0.3 ml of OPD as substrate for color development. After 30 min., color development was stopped by addition of 1 ml of 1N $H_2SO_4$, and the absorbance value at 492 nm was determined. The absorbance value was directly proportional to the amount of [HRPO]5-21-3 bound to the bead.

In the presence of competing anti-HIV I gp41 antibody in the serum sample, the amount of [HRPO]5-21-3 bound to the bead was significantly decreased. A cutoff value (c/o) was established as c/o = 0.5(NC-PC), where PC (positive control) is the assay absorbance value for an established anti-gp41 seropositive human serum, and NC (negative control) is the absorbance value for an established anti-gp41 seronegative serum. Test sera with values less than the c/o value were considered positive for anti-gp41 antibody because they blocked binding of [HRPO]5-21-3 to the target rp41, and sera with values above the c/o value were considered seronegative for anti-gp41 antibody. In addition, in order to account for daily fluctuations in the c/o value, the clinical data were expressed as sample:c/o ratios, where the assay $A_{492}$ value of the sample was divided by the daily assay c/o value. Seropositive samples exhibited ratios of <1.0, whereas seronegative samples exhibited ratios of ≥1.0.

B. Clinical Utility

The clinical utility of the Monoclonal Assay was assessed by comparing it to a similar assay (Abbott ENVACOR Assay) of established clinical efficacy ( Dawson et al., J. Infect. Dis. (Jan.,1988) 157:149-155 ; Frosner et al., Lancet (1987) i:159-160; Goudsmit et al., J. Infect. Dis. (1987) 155:558-560; and Habermehl et al., Infection (1987) 14:216). The configuration of the envelope (ENV) portion of the ENVACOR assay was nearly identical to the Monoclonal Assay except that the envelope assay employed human, polyclonal anti-HIV I IgG (purified from serum of an HIV I gp41 seropositive individual) as the HRPO-conjugated competitive probe (ENV Polyclonal Assay). Table 2 presents the results of several population studies comparing the Monoclonal and ENV Polyclonal Assays. The Monoclonal Assay was highly effective in detecting points of seroconversion in longitudinal studies, and the utility of the Monoclonal Assay was not affected by the geographic source of the specimen, nor by the disease state of the individual. In addition, the Monoclonal Assay was less susceptible to false negative results than the ENV Polyclonal Assay in the study (see footnote f).

Table 2.  Population Studies Comparing the Clinical Efficacy of Polyclonal (Abbott ENV Assay) vs. Monoclonal (Mab 5-21-3) Competitive Assays for Detection of Anti-HIV I gp41 Antibody.

| Source of Sera | No. of Individuals | No. of Serum Samples | Donor Status | ENV+ Mab+ | ENV- Mab- | ENV+ Mab- | ENV- Mab+ |
|---|---|---|---|---|---|---|---|
| Chicago USA | 120 | 152 | AIDS-28 | 38 | 0 | 0 | 0 |
| | | | ARC-10 | 16 | 0 | 0 | 0 |
| | | | ASYM-80 | 56 | 39 | [a]1 | 0 |
| | | | Other-2 | [b]1 | [c]1 | 0 | 0 |
| California USA | Unknown[d] | 19 | AIDS-10 | 9 | 1 | 0 | 0 |
| | | | LAS-10 | 10 | 0 | 0 | 0 |
| Berlin W. Germany[e] | 31 | 122 | AIDS-6 | 22 | 5 | 0 | 0 |
| | | | ARC-8 | 30 | 5 | 0 | [f]2 |
| | | | ASYM-9 | 20 | 11 | 0 | 0 |
| | | | Other-8 | 22 | 4 | 0 | [g]1 |
| W. Germany | Unknown[d] | 59 | AIDS-10 | 10 | 0 | 0 | 0 |
| | | | ARC-10 | 10 | 0 | 0 | 0 |
| | | | ASYM-30 | 30 | 0 | 0 | 0 |
| | | | Other-9 | 9 | 0 | 0 | 0 |
| Rome, Italy | 35 | 35 | Unknown | 15 | 20 | 0 | 0 |
| Zaire, Africa | 40 | 40 | Varied | [h]2 | [h]38 | 0 | 0 |
| Amsterdam, Netherlands[j] | 32 | 202 | High Risk | [k]132 | 65 | [l]5 | 0 |

[a]Asymptomatic male, on 9/25/85, tested Abbott EIA screening test negative, Mab assay negative, Abbott CORE (p24) test negative, and Abbott ENV positive.  All tests were negative on serum taken four months later.

[b]Female contact of a seropositive male.

[c]Young male of Mediterranean descent with Kaposi's sarcoma not associated with AIDS.

[d]Specimens randomly picked from the Abbott HTLV III EIA Screening Assay clinical trials, and donor status could reflect multiple samples from a few individuals.

[e]A longitudinal study.

[f]Serum samples which were Abbott EIA screening assay positive and Abbott CORE (p24) positive.

[g]Serum sample which was Abbott EIA screening assay positive and Abbott CORE (p24) negative.

[h]All sera were tested using the Abbott EIA screening assay, of which four were positive. Of those four, two were positive by the ENV and Mab tests, and two were negative by both tests. The remaining 36 sera were all negative by the screening assay and Mab assay, and are assumed to be ENV negative.

[j]A longitudinal, seroconversion study with blood samples drawn every 3 months up to the time of seroconversion. All samples were diluted 1:3 prior to assay due to small sera volumes.

[k]Includes 27 seroconversion points.

[l]All five sera were seroconversion points, three of which were Mab borderline and may have been positive using undiluted sera; the remaining two sera were discrepant.

Table 3 presents an analysis of the sensitivity and specificity of the 5-21-3 Monoclonal Assay based on the data presented in Table 2. A modified calculation of sensitivity and specificity also is presented, which accounts for assay variations, dilutions of clinical specimens, and false positives presented by the ENV Polyclonal Assay. Based on these data, the clinical utility of the Monoclonal Assay was firmly established, in that it detected greater than 99% of all HIV I gp41 seropositives detected by the Polyclonal Assay, had a false negative rate of less than 1%, and was less susceptible to false positives.

Table 3

| ENV Assay | Sensitivity and Specificity of the Monoclonal Competitive Assay Compared to the ENV Polyclonal Competitive Assay. | | | |
| --- | --- | --- | --- | --- |
| | Monoclonal Assay | | | |
| | Pos | | Neg | |
| Pos<br>Neg | (432)<br>[+] (3) | 437<br>1 | (6)<br>(189) | [*]2<br>190 |
| Sensitivity<br>Specificity | (98.6)<br>(98.4) | | | 99.5<br>99.5 |
| Original data presented in parentheses; modified data presented to the right of original data. | | | | |

[*]1 ENV false positive (Table 2, footnote a) is placed as ENV neg/Mab neg; 3 Mab borderline false negatives (Table 2, footnote 1) are placed as ENV pos/Mab pos: the remaining 2 ENV pos/Mab neg samples are two discrepant seroconversion points.

[+]2 ENV false negative samples (Table 2, footnote f) are placed as ENV pos/Mab pos; the remaining 1 ENV neg/Mab pos sample may represent a Monoclonal assay false positive (Table 2, footnote g).

The Polyclonal Assay has been evaluated by several laboratories and has been found to be equivalent to or superior over current blotting procedures or other EIA assays for detection of HIV I seropositive individuals (Dienhardt et al., Lancet (1987) Jan. 3:40; Denis et al., Lancet (1987) Feb. 7:324; Allain et al., Lancet (1986) Nov. 29:1233- 1236; Habermehl et al., supra; and Dawson et al., supra). Therefore based on the high degree of sensitivity of the Monoclonal Assay, compared to the Polyclonal Assay, it was inferred that the Monoclonal Assay also was equivalent to or superior over most other blotting or EIA assays.

Example 7

Mapping and Characterization of the gp41 Epitope Recognized by Monoclonal Antibody 5-21-3

A. Synthesis of Synthetic Peptides

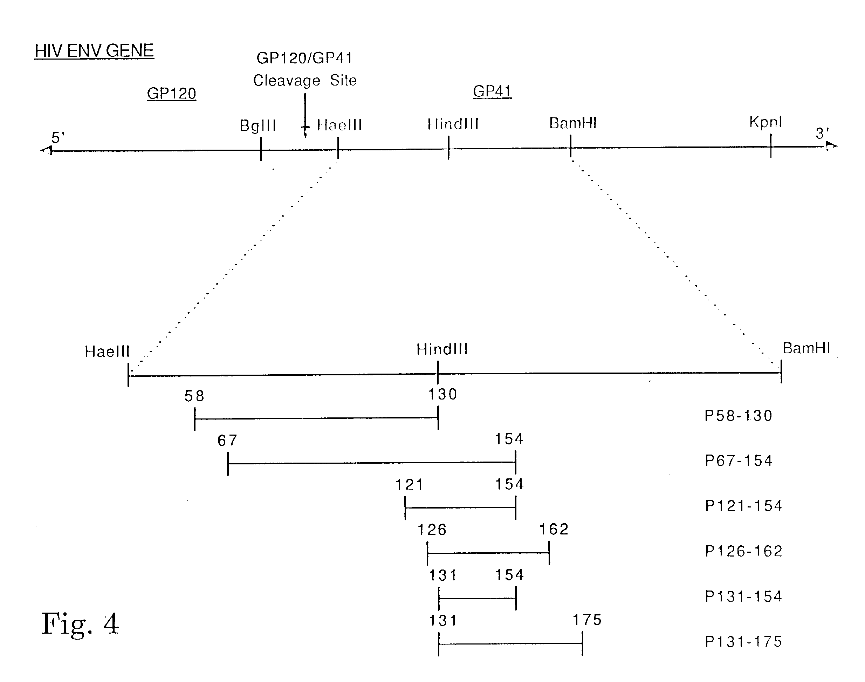

Fig. 4 illustrates the location of the synthetic peptides which were made in order to map the epitope recognized by monoclonal antibody 5-21-3. The synthesis of each peptide is outlined below:

1) Synthesis of the peptide corresponding to gp41 amino acids 121-154.

```
NH2-Asp-Arg-Glu-Ile-Asn-Asn-Tyr-Thr-Ser-Leu-Ile-His-Ser-Leu-Ile-Glu-Glu

-Ser-Gln-Asn-Gln-Gln-Glu-Lys-Asn-Glu-Gln-Glu-Leu-Leu-Glu-Leu-Asp-Lys-COOH
```

This polypeptide was assembled on a resin support by stepwise solid phase synthesis (starting with the carboxy-terminal residue). A procedure was employed similar to the one described by Barary and Merrifield (E. Gross and J. Meinehofer, Eds., The Peptides, vol. 2 [Academic Press, New York, 1980], p. 1284). A Boc-L-Arg(Tos)-OCH$_2$-Pam resin was transferred to a reaction vessel of an Applied Biosystems Synthesizer, Model 430A. Protected amino acids were coupled in a stepwise manner to the resin support by preformed symmetric anhydride chemistry, except, in the cases of arginine, asparagine, and glutamine addition, where the DCC/HOBT protocol described by Konig and Geiger (Chem. Ber. (1970) 103:788-798) was employed. All amino-terminal residues were protected by t-butyloxy carboxyl (t/BOC)-linkage, and side chains of various amino acid residues were protected by the following groups: Arg, Tos; Lys, 2-ClZ; Glu, OBZl; Thr, Bzl; Tyr, 2-BrZ.

The fully protected peptide-resin (0.5-1.0g) was allowed to swell in methylene chloride (CH$_2$Cl$_2$) for 5 minutes. The N-Boc protecting groups were removed using 60% trifluoroacetic acid (TFA/CH$_2$Cl$_2$) deprotection, CH$_2$Cl$_2$ washes, 10% N,N-diisopropylethylamine (DIEA/CH$_2$Cl$_2$) neutralization, and final CH$_2$Cl$_2$ washes. The resin was dried in vacuo. The peptide-resin thus obtained was treated with 9 ml of anhydrous hydrofluoric acid HF), to which 1 ml of p-cresol had been added, for 60 minutes at 0°C. The HF was distilled off in vacuo at 0°C. The cleaved, free peptide and resin were washed 3 times with 15 ml aliquots of diethyl ether. The peptide was then extracted three times with 15 ml of 40% aqueous acetic acid. The aqueous extracts were combined, washed three times with 10 ml aliquots of diethyl ether, and the aqueous layer was lyophilized to provide the crude peptide for purification.

The polypeptide was purified by reversed-phase HPLC on C$_4$ columns, employing gradients of 0.1% TFA/water (A) and 100% acetonitrile (B) as the solvent systems at a flow rate of 1 ml/min for the analytical column (Vydac-214-TP54, Vydac Separation Group, Hesperia, California) or 3 ml/min for the semi-preparative one (Vydac-214-TP510).

The gradient used was:

| 28% B | 1min | 28%B | 20min | 47%B | 1min | 28%B |
|-------|------|------|-------|------|------|------|

The polypeptide elution from the HPLC column was monitored at 225 nm and 280 nm. The composition of the polypeptide was confirmed by hydrolysis in 6 N hydrochloric acid (HCl)/0.3% phenol at 150°C for 2 hr in vacuo, and subsequently analyzed on a Beckman 6300 amino acid analyzer with a SICA 7000 A integration.

2) Synthesis of the peptide corresponding to gp41 amino acids 126-162.

```
NH2-Tyr-Asn-Tyr-Thr-Ser-Leu-Ile-His-Ser-Leu-Ile-Glu-Glu-Ser-Gln-Asn-Gln

-Gln-Glu-Lys-Asn-Glu-Gln-Glu-Leu-Leu-Glu-Leu-Asp-Lys-Trp-Ala-Asn

-Leu-Trp-Asn-Trp-Leu-COOH
```

This sequence of the peptide was assembled on the solid support by essentially the same procedure described above. The amino acid tryptophan was protected by the formyl (CHO) group. Double coupling protocols were used for amino acids underlined in the sequence shown above. The desired peptide was deprotected and cleaved off as described for the first peptide except that the peptide-resin was treated with a mixture of 8.5 ml HF, 1.0 ml of p-cresol and 0.5 ml ethanedithiol. The cleaved peptide was extracted using 15% and 40% aqueous acetic acid. The crude peptide so obtained was analyzed and purified on a C$_4$ reversed-phase column as described above except the gradient used was:

14

| 35%B | 1 min | 35%B | 20 min concave curve | 70%B | 2 min linear | 35%B |
|---|---|---|---|---|---|---|

3) Synthesis of peptide corresponding to gp41 amino acids 131-154.

```
NH2-Ile-His-Ser-Leu-Ile-Glu-Glu-Ser-Gln-Asn-Gln-Gln-Glu-Lys-Asn-Glu-
Gln-Glu-Leu-Leu-Glu-Leu-Asp-Lys-COOH
```

This peptide was assembled, cleaved, and purified as described for the first peptide, except that the following gradient was used for analysis and purification:

| 15%B | 1 min | 15%B | 20 min linear | 40%B | 2 min | 40%B | 2 min linear | 15%B |
|---|---|---|---|---|---|---|---|---|

4) Synthesis of peptide corresponding to gp41 amino acids 67-154.

```
NH2-Ala-Arg-Ile-Leu-Ala-Val-Glu-Arg-Tyr-Leu-Lys-Asp-Gln-Gln-Leu-Leu-
Gly-Ile- Trp-Gly-Cys-Ser-Gly-Lys-Leu-Ile-Cys-Thr-Thr-Ala-Val-Pro-Trp-
Asn-Ala-Ser-Trp- Ser-Asn-Lys-Ser-Leu-Glu-Gln-Ile-Trp-Asn-Asn-Met-Thr-
Trp-Met-Glu-Trp-Asp-Arg-Glu-Ile-Asn-Asn-Tyr-Thr-Ser-Leu-Ile-His-Ser-
Leu-Ile-Glu-Glu-Ser-Gln-Asn-Gln-Gln-Glu-Lys-Asn-Glu-Gln-Glu-Leu-Leu-
Glu-Leu-Asn-Lys-COOH
```

This peptide was assembled on the solid support as described in (1) above with the following differences. A single coupling protocol was used for assembling amino acids from 154 to 143 except for asparagine and glutamine which were coupled twice. Double coupling protocols were used for all subsequent amino acids from 142 to 67. During this synthesis, 0.45 g and 0.60 g of the resin, respectively, were taken out after the addition of amino acids aspartic acid at position 121 and serine at position 104. The integrity of the assembled peptide was confirmed by solid phase sequencing (11 to 18 cycles) during synthesis. Specifically, the peptide-resin was sequenced (i) after adding amino acid aspartic acid at position 121 (121-154), (ii) after adding amino acid leucine at 82 (82-154), and (iii) at the end of the synthesis (67-154). In all cases, the overwhelming major sequence observed was the desired one, with minor amounts of deletion sequences.

The desired peptide was deprotected and cleaved off the resin using the "low high HF" procedure as described by Tam et al., J. Am. Chem. Soc. (1983) 105:6442-6455. After evaporation of HF, the reaction mixture was washed with ether followed by extraction of the free peptide with 40% aqueous acetic acid. The resultant acetic acid extract, which was turbid with precipitates, was centrifuged to obtain an insoluble pellet. The supernatant was lyophilized to obtain a white crude peptide. The insoluble pellet was dried in a lyophilizer overnight to also yield crude peptide.

The crude peptide (67-154) obtained above was dissolved in an 8 M urea, 50 mM Tris-HCl pH 8.2, buffer containing 50 mM dithiothreitol (DTT). This solution was incubated at 42°C for 1 hr, and then dialyzed in a spectrapor membrane (cut-off range 6000-8000) against the following buffers:

(i) 4 M urea, 50 mM Tris-HCl, pH 8.2, 50 mM DTT for 16-18 hr;
(ii) 3 M urea, 50 mM Tris-HCl, pH 8.2, 50 mM DTT for 48 hr;
(iii) 50 mM Tris-HCl, pH 8.1, for 6-10 hr with 4 exchanges of fresh buffer.

The stock solution of the peptide thus obtained was reduced with 50 mM DTT at 40°C for 90 minutes. The solution was brought to room temperature, and then dialyzed in a spectrapor membrane (cutoff 6500-8000) against a 0.1 M ammonium acetate buffer, pH 8.1, for 48 hr. The buffer was changed twice. After a total of 72 hr of dialysis, the peptide solution was diluted 3-fold with 0.1 M ammonium acetate buffer, pH 8.1, and allowed to stand in air for 48 hr. A UV spectrum of this peptide solution in water showed a maxima at 276 nm with a shoulder at 289 nm. The peptide was further purified on a reversed-phase $C_4$ column, and then analyzed as described above, using the following gradient:

| 30%B | 1 min | 30%B | 20 min | 65%B | 1 min | 30%B |
|---|---|---|---|---|---|---|

5) Synthesis of peptide corresponding to gp41 amino acids 58-130.

```
NH2-Thr-Val-Trp-Gly-Ile-Lys-Glu-Leu-Gln-Ala-Arg-Ile-Leu-Ala-Val-Glu-
Arg-Tyr-Leu-Lys-Asp-Gln-Gln-Leu-Leu-Gly-Ile-Trp-Gly-Cys-Ser-Gly-Lys-Leu-
Ile-Cys-Thr-Thr-Ala-Val-Pro-Trp-Asn-Ala-Ser-Trp-Ser-Asn-Lys-Ser-Leu-Glu-
Gln-Ile-Trp-Asn-Asn-Met-Thr-Trp-Met-Glu-Trp-Asp-Arg-Glu-Ile-Asn-Asn-Tyr-
Thr-Ser-Leu-COOH
```

6) Synthesis of peptide corresponding to gp41 amino acids 131-175.

```
NH2-Ile-His-Ser-Leu-Ile-Glu-Glu-Ser-Gln-Asn-Gln-Gln-Glu-Lys-Asn-Glu-
Gln-Glu-Leu-Leu-Glu-Leu-Asp-Lys-Trp-Ala-Asn-Leu-Trp-Asn-Trp-Leu-Asn-Ile-
Thr-Asn-Trp- Leu-Trp-Tyr-Ile-Lys-Leu-Phe-Ile-COOH
```

Synthesis and purification of these peptides proceeded using the methods outlined above.

B. Epitope Mapping of Antibody 5-21-3 by Immuno-Dot Blotting Using Synthetic Peptides

The western Blot procedure described in Example 1 was employed for development of immuno-dot blots using synthetic peptides, except that synthetic peptides were fixed to nitrocellulose by direct application using Drummond lambda pipettes, and air dried prior to blocking with 20% fetal calf serum in PBS (pH 7.4). Monoclonal IgG 5-21-3, and a second anti-HIV I gp41 monoclonal antibody (IgG) 10-15-63 (which recognizes an epitope distinct and separate from the epitope recognized by 5-21-3), were purified from mouse ascites using Protein A-Sepharose, and employed at the concentrations indicated.

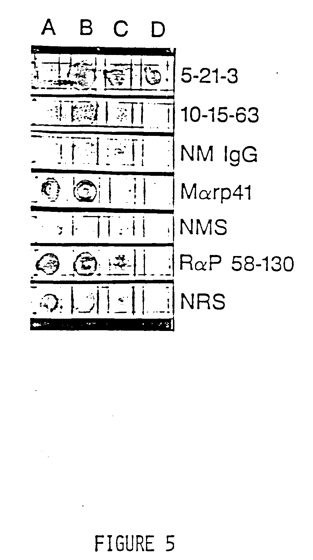

Immuno-dot blot results are illustrated in Figure 5 where synthetic peptides were spotted onto nitrocellulose as follows:(A) 10 ug P58-130, (B) 10 ug P67-154,(C) 6 ug P121-154 and (D) 9 ug of P126-162. The strips were reacted with 25 ug/ml monoclonal antibody 5-21-3, 25 ug/ml monoclonal antibody 10-15-63, 25 ug/ml normal mouse IgG, 2% mouse (M) anti-rp41 serum, 2% normal mouse serum (NMS), 2% Rabbit (R) anti-P58-130 serum, or 2% normal rabbit serum (NRS). Dot blot results (Fig. 5) clearly demonstrated that antibody 5-21-3 did not react with P58-130. In sharp contrast, addition of amino acids 131-154 to the carboxy end to form P67-154 (which lacks 10 amino acids on the amino end) produced a very strong reaction with antibody 5-21-3. Strong reactivities against both P58-130 and P67-154 were detected using rabbit anti-P58-130 serum and mouse anti-rp41 serum, demonstrating that the peptides were present on the nitrocellulose. Little or no reactivity against either peptide could be demonstrated using monoclonal antibody 10-15-63, normal mouse IgG, normal mouse serum, or normal rabbit serum. Under conditions employing mild washing procedures, additional 5-21-3 reactivity was observed against the smaller P126-162, and a faint, diffuse reaction against P121-154. Because smaller peptides may not bind tightly to nitrocellulose, reactivity of 5-21-3 by dot blot against smaller peptides, for example P121-154, P131-154, and P131-175 (not shown) was difficult to demonstrate. However, it is clear that addition of amino acids 131-154 to a larger peptide(67-154) provided an epitope recognized by 5-21-3, and that no reactivity was demonstrated against a similar peptide (P58-130) which lacked amino acids 131-154.

C. Radiolabeling of Synthetic Peptides

Synthetic peptides were radiolabeled with[125]I (Amersham) by the chloramine T method (W.M. Hunter, "Radioimmunoassay" in D.M. Weir, Ed., Handbook of Experimental Immunology, [Blackwell Scientific Publications, 1973]). Generally, 2-6 ul of 100 mCi/ml $^{125}$I were added to 10-50 ug of peptide in PBS, pH 7.4. Approximately 10 ug of freshly prepared (1 mg/ml in PBS) chloramine T were added to the mixture maintained at 4°C. After 5 min., [125I]peptide was separated from free $^{125}$I by passage of the reaction mixture through a 3 ml (bed volume) column of Biogel P-6 or P-2, as required. Fractions (200 ul each) containing [125I]peptide were pooled and diluted into PBS containing 20% fetal calf serum. Rough estimations of[125]I cpm bound to peptide were made by acid precipitation. An aliquot of labeled peptide was diluted into 1 ml of ice cold 50% trichloroacetic acid (TCA) overlaying a 0.45 um filter (made of mixed cellulose esters) set on a scintered glass filter connected to a vacuum apparatus. Precipitated protein was fixed onto the filter by application of vacuum, and filters were washed with ice-cold 50% TCA. Background cpm adhering non-specifically to the filter were estimated by repeating the procedure using a non-precipitating buffer. Amounts of peptide, $^{125}$I, chloramine T, and composition of column eluting buffer were subject to change depending on conditions required to maintain solubility of each peptide.

D. Epitope Mapping of 5-21-3 by Immunoprecipitation of Radiolabeled Synthetic Peptides

Synthetic peptides radiolabeled with[125]I were diluted into PBS (pH 7.4) containing 0.1% Triton X-100 (or other detergents where indicated) and 1.0% BSA, to yield approximately $2.5\text{-}5 \times 10^5$ acid-precipitable cpm per 0.1 ml. Purified monoclonal IgG 5-21-3 and 10-15-63 were employed in the assay. Monoclonal antibodies were diluted into PBS containing 0.1% Triton X-100 (or other detergents where indicated), 1.0% BSA, and 2% normal mouse serum as a carrier immunoglobulin. Immune rabbit serum (anti-P58-130) was diluted appropriately into the same buffer except that normal rabbit serum (2%) was substituted for normal mouse serum as the carrier immunoglobulin. A reaction mixture comprising 0.1 ml [125I] peptide ($5 \times l0^5$ cpm/0.1 ml) and 0.1 ml diluted antibody was incubated 1 hr at 40°C, followed by addition of 0.1 ml of a precipitating antibody, either goat anti-mouse IgG, or goat anti-rabbit IgG, corresponding to the primary antibody. The reaction mixture was incubated 1 hr at 40°C and then overnight at 4°C. The precipitate was collected by centrifugation (2,000 x g, 5 min., 20°C), washed once in PBS containing 0.1% Triton X-100 and 1.0% BSA, collected by centrifugation, and counted in an LKB 128 gamma counter. Precipitation assays employing Protein A-Sepharose as the precipitating reagent were performed in a similar manner, except that normal sera were omitted from the reaction mixtures.

In agreement with the dot blots discussed above, antibody 5-21-3 was unable to bind P58-130 (see Table 4), indicating that the amino acids which form the epitope are not present in this peptide. In sharp contrast, and in agreement with the dot blot data, addition of amino acids 131-154 to the carboxy end of P58-130 to form P67-154, produced an epitope which was readily bound by the 5-21-3 antibody. Antibody 5-21-3 was unable to bind P131-154 which contains only those amino acids which form the epitope. However, the addition of flanking sequences, 10 amino acids to the amino end of 131-154 to form P121-154, as well as 5 and 8 amino acids to the amino and carboxy ends, respectively, to form P126-162, provided an epitope which was recognized by 5-21-3. Little or no binding activity by 5-21-3 against P131-175 was detected. These data indicated that the correct linear amino acid sequence (P131-154) was not sufficient for recognition and binding of 5-21-3, but rather, additional sequence either 5′ (amino end) of amino acid 131 or 3′ (carboxy end) of amino acid 154, were required to put the linear sequence in the proper antigenic conformation for recognition by 5-21-3. For all peptides, specific binding using monoclonal 10-15-63 could not be detected. In addition, rabbit anti-P58-130 was a strong precipitator of P58-130, P67-154, and P121-154, indicating the presence of precipitable peptide.

Table 5 illustrates the effect of antibody concentration on binding activity of 5-21-3. Clearly, while as little as 1 ug/ml of 5-21-3 bound significant amounts of P67-154, at least 100 ug/ml of the antibody was required to demonstrate even low level reactivity against the smaller peptides P121-154 and P126-162, and reactivity against P131-154 could not be demonstrated. These data indicate that 5-21-3 may have a much greater affinity for its epitope when the epitope is presented as part of a larger peptide, as opposed to identical sequence presented as a much shorter peptide.

Table 4.  Precipitation of [$^{125}$I] Synthetic Peptides by Monoclonal Antibody 5-21-3.[a]

| [$^{125}$I]PEPTIDE | SPECIFIC PRECIPITATION (CPM)[b] | | |
|---|---|---|---|
| | 5-21-3 | 10-15-63 | R·P58-130 |
| P58-130[c] | 0 (<1) | 0 (<1) | 286,722 (32) |
| P67-154[d] | 479,207 (16) | 9713 (1.3) | 479,343 (4.4) |
| P131-154[d] | 970 (1.1) | 0 (<1) | 0 (<1) |
| P121-154[d] | 40,717 (31) | 371 (1.3) | 310,419 (24) |
| P126-162[d] | 11,540 (3.4) | 0 (<1) | 0 (<1) |
| P131-175[d] | 317 (1.6) | 188 (1.4) | 0 (<1) |

[a]Monoclonal antibodies 5-21-3 and 10-15-63 (anti-gp41 recognizing an epitope distinct and separate from that recognized by 5-21-3) were purified from mouse ascites and used at 10 ug/0.2 ml reaction mixture.  Rabbit (R) anti-P58-130 serum was used at 20 ul/0.2 ml reaction mixture.

[b]Specific precipitation was calculated as (specific cpm − non-specific cpm), where non-specific cpm was determined using normal mouse IgG (10 ug/0.2 ml) or normal rabbit serum (20 ul/0.2 ml).  Numbers in parentheses represent the x-fold increase of specific precipitation (using 5-21-3, 10-15-63, or R anti-P58-130) over non-specific precipitation (using normal mouse IgG or normal rabbit serum).  Between 250,000 and 500,000 trichloroacetic acid precipitable cpm of iodinated peptides were routinely employed per reaction mixture.

[c]Precipitation of P58-130 was performed using PBS buffer (pH 7.4) containing 0.1% Triton X-100, 1.0% sodium deoxycholate, and 0.1% SDS.  Immune complexes were collected using Protein A- Sepharose.  The detergent-rich buffer was chosen in order to retain solubility of P58-130.

[d]Precipitation of these peptides was performed using PBS buffer (pH 7.4) containing 0.1% Triton X-100, 1.0% bovine serum albumin, and 1.0% (final concentration) normal mouse or rabbit serum as carrier protein.  Immune complexes were precipitated using a second antibody, either goat anti-mouse IgG, or goat anti-rabbit IgG.

Table 5. Effect of 5-21-3 Antibody Concentration on Binding of Synthetic Peptides.[a]

SPECIFIC PRECIPITATION (CPM)[b]

| [$^{125}$I]PEPTIDE | 5-21-3 (ug/ml) | | | 10-15-63 (ug/ml) | | |
|---|---|---|---|---|---|---|
| | 1.0 | 10.0 | 100.0 | 1.0 | 10.0 | 100.0 |
| P67-154 | 165,781 (3.6) | 441,887 (8.5 | 659,045 (8.0) | 0 (0) | 0 (0) | 0 (0) |
| P121-154 | 0 (0) | 1,113 (2.3) | 18,984 (13) | 0 (0) | 0 (0) | 0 (0) |
| P126-162 | 0 (0) | 295 (1.1) | 4,924 (1.6) | 0 (0) | 0 (0) | 0 (0) |
| P131-154 | 0 (0) | 0 (0) | 0 (0) | 0 (0) | 0 (0) | 112 (1.1) |

[a]Precipitation of these peptides was performed using PBS buffer (pH 7.4) containing 0.1% Triton X-100, 1.0% bovine serum albumin, and 1.0% (final concentration) normal mouse serum as carrier protein. Immune complexes were precipitated using goat anti-mouse IgG. [b]Monoclonal antibodies 5-21-3 and 10-15-63 (anti-gp41) were purified from mouse ascites. The values (ug/ml) given for each antibody represent the final concentration of the antibody in the reaction mixture. Specific precipitation was calculated as (specific cpm - non-specific cpm), where non-specific cpm was determined using normal mouse IgG. Numbers in parentheses represent the x-fold increase of specific precipitation using 5-21-3 or 10-15-63, over non-specific precipitation using normal mouse IgG.

E. Epitope Mapping Using Synthetic Peptides to Inhibit Binding of 5-21-3 to rp41

Additional evidence for the conformational nature of the 5-21-3 epitope was obtained using binding inhibition studies, in which synthetic peptides were ranked for their ability to inhibit binding of 5-21-3 to rp41. First, the Fab dimer of an anti-gp41 monoclonal antibody was prepared by conventional methods (Goding et al., supra). Then 100 ul of a 1 mg/ml solution of the Fab dimer in bicarbonate buffer (pH 9.6) was coated onto Immulon II microtiter plates for 1 hr at 37°C. After washing with PBS containing 0.05% Tween 20, the plates were overcoated with 200 ul of 1.0% ovalbumin in bicarbonate buffer (pH 9.6) for 1 hr at 30°C. After washing, 100 ul of rp41 enriched E. coli lysate (as described in Example 3), diluted appropriately in PBS containing 0.05% Tween 20, were allowed to bind to the Fab dimer for 2 hr at 30°C. The plates were washed and stored at 4°C.

Intact monoclonal antibody 5-21-3 IgG (purified from tissue culture fluid supporting growth of the cloned cell line 5-21-3) was titrated against the bound rp41 on the plate, and an antibody concentration was selected which yielded an $A_{492}$ value of 2.0-3.0. Usually, 0.4 pmol (0.06 ug) of 5-21-3 IgG per microtiter well was employed in these assays. Stock solutions of 5-21-3 IgG were prepared in PBS containing 0.1% Triton X-100 and 0.1% SDS. Dilutions of synthetic peptides were made in the same buffer (SDS was included in these experiments to keep peptides soluble at high concentrations).

The binding inhibition assay was performed by combining 0.15 ml of 1.2 ug/ml 5-21-3 antibody stock solution with 0.15 ml of peptide at a selected peptide concentration, and incubating the reaction mixture 1 hr at room temp. Then 0.1 ml aliquots of each reaction mixture were added to each of two wells in the microtiter tray. After a 1 hr incubation at room temp., the plates were washed, and HRPO conjugated Goat anti-mouse IgG Fc was added for 1 hr at room temp. The plates were washed, and addition of OPD for color development identified the relative amounts of 5-21-3 bound to rp41 in the microtiter wells. The reaction was stopped by addition of 100 ul of 1N $H_2SO_4$, and the plates were read on a Dynatech II microtiter plate reader.

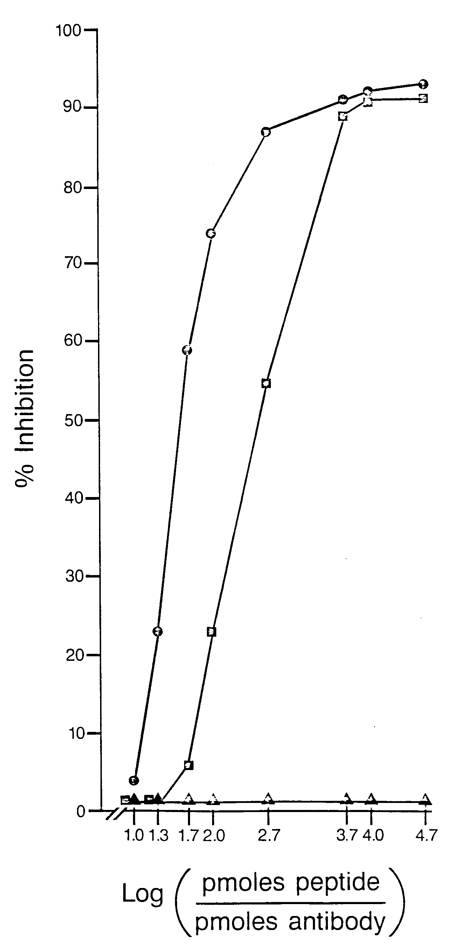

Consistent with previous dot blot and immunoprecipitation results, Fig. 6 demonstrates the lack of reactivity of P58-130 with 5-21-3. There was no detectable inhibition of 5-21-3 binding to rp41 when P58-130 was employed as the inhibitor. In sharp contrast, P67-154, which contains the additional carboxy-terminal amino acids 131-154, was a potent inhibitor of binding activity. In addition, P121-154 also was a potent inhibitor of binding, although considerably more peptide was required to elicit 50% inhibition, compared to P67-154.

Table 6 ranks the synthetic peptides for their ability to block binding activity of antibody 5-21-3. Consistent with the previous data, P67-154 was the most potent inhibitor, but was considerably less effective than native antigen (rp41). In addition, although P131-154 contains the same linear sequence of amino acids as the carboxy end of P67-154, inhibition of 5-21-3 with this peptide could not be demonstrated even up to a 50,000-fold molar excess of peptide. Furthermore, the addition of a flanking sequence, consisting of 10 amino acids 5′ (amino end) to amino acid 130 to produce P121-154, restored binding inhibition activity by at least 20-65 fold, although P121-154 was still 20-fold less active than P67-154. The binding inhibition activity of P126-162, which contains 5 and 8 additional amino acids 5′ (amino end) and 3′ - (carboxy end) of amino acids 130 and 154, respectively, was roughly equivalent to that of P121-154. Importantly, P131-175, which contains no additional amino acids 5′ of amino acid 131, was a definite inhibitor of 5-21-3 binding activity, although only at high concentrations. Flanking sequence 3′ (carboxy end) of amino acid 154 in P131-175, apparently contributes to the antigenic conformation of the 131-154 epitope, as does sequence 5′ (amino end) of amino acid 131 (for example, P121-154). More importantly, amino acids on the 5′ (amino) side of amino acid 131 are not absolutely required in order to identify the location of the amino acids which will ultimately form the native epitope recognized by the monoclonal antibody when sufficient flanking sequences are present.

Table 6

| Relative Concentrations of Synthetic Peptides Required to Inhibit Binding of Antibody 5-21-3 to rp41. | |
|---|---|
| Inhibitor (Peptide) | Molar Ratio[a] |
| rp41 | 9.5 |
| P67-154 | 72.7 |
| P131-154 | >50,000 |
| P121-154 | 1,494 |
| P126-162 | 1,585 |
| P131-175 | 18,815 |
| P194-220[b] | >50,000 |

[a]Ratios are expressed as pmoles peptide/pmoles 5-21-3, at 50% inhibition of 5-21-3 binding; each number represents the mean of two experiments.

[b]This peptide, used as a control, was synthesized from a region outside the epitope of 5-21-3.

Results of the immuno-dot blot, immunoprecipitation, and binding inhibition assays, all indicate that the amino acids which form the epitope recognized by monoclonal antibody 5-21-3 lie 3′ of amino acid 130 (the HindIII site), and 5′ of amino acid 154. Proper conformation of the amino acids comprising this region is essential to form the epitope recognized by antibody 5-21-3. The conformation-dependent nature of the epitope is evidenced by the marked influence of flanking sequences (5′ of amino acid 131 or 3′ of amino acid 154) on binding of antibody 5-21-3 to peptides containing sequence 131-154. Further, linear sequences comprising the region of interest, amino acids 131-154, do not by themselves form the epitope recognized by the 5-21-3 antibody. Thus, the linear sequence is not antigenic, and by inference, does not form the antigenic determinant found in the native protein which was used as the immunogen for production of B cells secreting 5-21-3 antibody. By analogy, human antibodies elicited by native gp41 which compete with antibody 5-21-3, also must recognize the native antigenic determinant which is conformation dependent, and is identical to or indistinguishable from the epitope recognized by antibody 5-21-3. Proper evaluation of human sera which recognize the 5-21-3 epitope (as determined by their ability to compete with 5-21-3) by methods other than competition with 5-21-3, must take into account that short linear peptides or cloned gene fragments which do not contain sufficient sequence cannot present the native epitope. Recombinant proteins which utilize and begin at the Hind III site in gp41 will not form the complete native epitope, nor can the fully antigenic native epitope be detected using such recombinant proteins. Similarly, synthetic peptides claiming linear or conformational epitopes which comprise the sequence between amino acids 131-154 without sufficient flanking sequences cannot represent the fully antigenic, native epitope. Apparently, native or near full-length recombinant gp41 may be required to form the native epitope which is recognized by 5-21-3 and which is clinically useful as a serological target.

Changes and modifications may be conceived and practiced without departing from the scope of the invention and it is understood that such equivalent embodiments are intended to be included herein.

**Claims**

**Claims for the following Contracting States : AT, BE, CH, DE, FR, GB, IT, LI, NL**

1. A monoclonal antibody characterized by its specificity for an epitope on HIV I gp41 formed by a first sequence of amino acids

```
Ile-His-Ser-Leu-Ile-Glu-Glu-Ser-Gln-Asn-Gln-Gln-Glu-Lys-Asn-Gl
u-Gln-Glu-Leu-Leu-Glu-Leu-Asp-Lys
```

with at least one flanking second amino acid sequence of at least 5 amino acids in length either 3' to the carboxy terminus or 5' to the amino terminus of said first sequence, said flanking sequence having an amino acid sequence corresponding to native HIV I gp41, wherein said flanking sequence places said first sequence into proper antigenic conformation.

2. A hybridoma antibody-producing cell line that produces the monoclonal antibody of Claim 1.

3. The cell line of claim 2 which comprises a cell hybrid of a mouse spleen cell immunized with HIV I fused to myeloma cell line SP2/0.

4. A method for detecting a marker indicative of exposure to HIV I in a sample comprising forming an antibody/antigen complex between the epitope on HIV I gp41 formed by a first sequence of amino acids

```
Ile-His-Ser-Leu-Ile-Glu-Glu-Ser-Gln-Asn-Gln-Gln-Glu-Lys-Asn-Gl
u-Gln-Glu-Leu-Leu-Glu-Leu-Asp-Lys
```

with at least one flanking second amino acid sequence of at least 5 amino acids in length either 3' to the carboxy terminus or 5' to the amino terminus of said first sequence, said flanking sequence having an amino acid sequence corresponding to native HIV-I gp41 and an antibody specific for that epitope; and detecting the presence or amount of the antibody/antigen complex formed.

5. The method of claim 4 wherein the antibody/antigen complex is formed in an immunometric, competitive, sandwich, or agglutination assay format.

6. The method of claim 5 wherein the antibody is a monoclonal antibody.

7. An immunoassay method for determining the presence or amount of antibody to HIV I gp41 which may be present in a test sample, comprising: incubating the test sample with a solid phase-bound binding material containing a target epitope having the immunological properties of the epitope on HIV I gp41 formed by a first sequence of amino acids

```
Ile-His-Ser-Leu-Ile-Glu-Glu-Ser-Gln-Asn-Gln-Gln-Glu-Lys-Asn-Gl
u-Gln-Glu-Leu-Leu-Glu-Leu-Asp-Lys
```

with at least one flanking second amino acid sequence of at least 5 amino acids in length either 3' to the carboxy terminus or 5' to the amino terminus of said first sequence, said flanking sequence having an amino acid sequence corresponding to native HIV I gp41, and with a probe antibody which specifically binds to the target epitope of the binding material; and determining the presence or amount of the probe antibody bound or unbound to the binding material as an indication of the presence or amount of antibody to HIV I gp41 in the test sample.

8. The immunoassay method of claim 7, wherein said binding material comprises partially purified HIV I, native HIV I gp41, or full-length recombinant-derived gp41.

9. A diagnostic kit for detection of exposure to HIV I comprising the monoclonal antibody of claim 1 as a reagent.

10. The process of producing a monoclonal antibody of claim 1 comprising the steps of:
    a) immunizing a mammal with HIV-I antigen;
    b) demonstrating specific anti-HIV-I antibody production by said immunized mammal; and
    c) fusing the spleen cells of said immunized mammal with a myeloma cell line thereby forming a hybridoma that which secretes anti-HIV-I antibody, wherein said antibody is characterized by its specificity for an epitope on HIV I gp41 formed by a first sequence of amino acids

```
Ile-His-Ser-Leu-Ile-Glu-Glu-Ser-Gln-Asn-Gln-Gln-Glu-Lys-Asn-Glu-Gln-Glu-Leu-
Leu-Glu-Leu-Asp-Lys
```

with at least one flanking second amino acid sequence of at least 5 amino acids in length either 3' to the carboxy terminus or 5' to the amino terminus of said first sequence, said flanking sequence having an amino acid sequence corresponding to native HIV I gp41, wherein said flanking sequence places said first sequence into proper antigenic conformation.

11. The process of Claim 10, wherein said hybridoma comprises a cell hybrid of a mouse spleen cell immunized with HIV I fused to myeloma cell line SP2/0.

**Claims for the following Contracting State : ES**

1. The process of producing a monoclonal antibody, comprising the steps of
    a) immunizing a mammal with HIV-I antigen;
    b) demonstrating specific anti-HIV-I antibody production by said immunized mammal; and
    c) fusing the spleen cells of said immunized mammal with a myeloma cell line thereby forming a hybridoma that which secretes anti-HIV-I antibody, wherein said antibody is characterized by its specificity for an epitope on HIV I gp41 formed by a first sequence of amino acids

```
Ile-His-Ser-Leu-Ile-Glu-Glu-Ser-Gln-Asn-Gln-Gln-Glu-Lys-Asn-Glu-Gln-Glu-Leu-
Leu-Glu-Leu-Asp-Lys
```

with at least one flanking second amino acid sequence of at least 5 amino acids in length either 3' to the carboxy terminus or 5' to the amino terminus of said first sequence, said flanking sequence having an amino acid sequence corresponding to native HIV I gp41, wherein said flanking sequence places said first sequence into proper antigenic conformation.

22

2. The process of Claim 2, wherein said hybridoma comprises a cell hybrid of a mouse spleen cell immunized with HIV I fused to myeloma cell line SP2/0.

3. A method for detecting a marker indicative of exposure to HIV I in a sample, comprising:
   forming an antibody/antigen complex between the epitope on HIV gp41 formed by a first sequence of amino acids

Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys

with at least one flanking second amino acid sequence of at least 5 amino acids in length either 3' to the carboxy terminus or 5' to the amino terminus of said first sequence, said flanking sequence having an amino acid sequence corresponding to native HIV I gp41 and an antibody specific for that epitope; and
   detecting the presence or amount of the antibody/antigen complex formed.

4. The method of Claim 3, wherein the antigen/antibody complex is formed in an immunometric, competitive, sandwich or agglutination assay format.

5. The method of Claim 4, wherein the antibody is a monoclonal antibody.

6. An immunoassay method for determining the presence or amount of antibody to HIV I gp41 which may be present in a test sample, comprising:
   incubating the test sample with a solid phase-bound binding material containing a target epitope having the immunological properties of the epitope on HIV I gp41 formed by a first sequence of amino acids

Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys

with at least one flanking second amino acid sequence of at least 5 amino acids in length either 3' to the carboxy terminus or 5' to the amino terminus of said first sequence, said flanking second sequence having an amino acid sequence corresponding to native HIV I gp41, and with a probe antibody which specifically binds to the target epitope of the binding material, and
   determining the presence or amount of the probe antibody bound or unbound to the binding material as an indication of the presence or amount of antibody to HIV I gp41 in the test sample.

7. The immunoassay method of claim 6, wherein said binding material comprises partially purified HIV I, native HIV I gp41 antigen or full-length recombinant-derived gp41.

**Patentansprüche**
**Patentansprüche für folgende Vertragsstaaten : AT, BE, CH, DE, FR, GB, IT, LI, NL**

1. Monoklonaler Antikörper, gekennzeichnet durch seine Spezifität gegen ein Epitop auf HIV I gp41, welches von einer ersten Sequenz von Aminosäuren

Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys

gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die flankierende Sequenz eine Aminosäuresequenz hat, die natürlichem HIV I gp41 entspricht, und wobei die flankierende Sequenz die erste Sequenz in die richtige antigenische Konformation versetzt.

2. Antikörper-produzierender Hybridzellstamm, der den monoklonalen Antikörper nach Anspruch 1 produziert.

3. Zellstamm nach Anspruch 2, der eine Hybridzelle aus einer mit HIV I immunisierten Mäusemilzzelle umfaßt, die mit dem Myelomzellstamm SP2/0 verschmolzen ist.

4. Verfahren zum Nachweis einer Markierung in einer Probe, die den Kontakt mit HIV I anzeigt, folgendes umfassend:
   - Bildung eines Antikörper/Antigen-Komplexes zwischen
       dem Epitop auf HIV I gp41, welches von einer ersten Sequenz von Aminosäuren

```
Ile-His-Ser-Leu-Ile-Glu-Glu-Ser-Gln-
Asn-Gln-Gln-Glu-Lys-Asn-Glu-Gln-Glu-Leu-Leu-Glu-Leu-Asp-Lys
```

   gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die flankierende Sequenz eine Aminosäuresequenz hat, die natürlichem HIV I gp41 entspricht,
       und einem gegen dieses Epitop spezifischen Antikörper;
   - und den Nachweis der Gegenwart oder Menge des gebildeten Antikörper/Antigen-Komplexes.

5. Verfahren nach Anspruch 4, wobei der Antikörper/Antigen-Komplex in einem immunometrischen, Konkurrenz-, Sandwich-, oder Agglutinations-Assayformat gebildet wird.

6. Verfahren nach Anspruch 5, wobei der Antikörper ein monoklonaler Antikörper ist.

7. Immunoassayverfahren zur Bestimmung der Anwesenheit oder der Menge von Antikörpern gegen HIV I gp41, die in der Testprobe vorhanden sein kann, folgendes umfassend:
   - Inkubation der Testprobe
       mit einem festphasenverankerten Bindungsmaterial, welches ein Zielepitop enthält, das die immunologischen Eigenschaften des Epitops auf HIV I gp41 hat, welches von einer ersten Sequenz von Aminosäuren

```
Ile-His-Ser-Leu-Ile-
Glu-Glu-Ser-Gln-Asn-Gln-Gln-Glu-Lys-Asn-Glu-Gln-Glu-Leu-Leu-
Glu-Leu-Asp-Lys
```

   gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die flankierende Sequenz eine Aminosäuresequenz hat, die natürlichem HIV I gp41 entspricht,
       und mit einem Sondenantikörper, der spezifisch an das Zielepitop des Bindungsmaterials bindet;
   - Bestimmen der Anwesenheit oder Menge an Sondenantikörper, der an das Bindungsmaterial gebunden oder welcher ungebunden ist, als Anzeige für die Anwesenheit oder Menge des Antikörpers gegen HIV I gp41 in der Testprobe.

8. Immunoassayverfahren nach Anspruch 7, wobei das Bindungsmaterial teilweise gereinigtes HIV I, natürliches HIV I gp41 oder rekombinant-abgeleitetes gp 41 von voller Länge umfaßt.

24

**9.** Diagnostisches Kit zum Nachweis des Kontaktes mit HIV I, welches den monoklonalen Antikörper nach Anspruch 1 als Reagens umfaßt.

**10.** Verfahren zur Herstellung eines monoklonalen Antikörpers nach Anspruch 1, welches folgende Schritte umfaßt:

a.) Immunisieren eines Säugers mit HIV I-Antigen,

b.) Demonstrieren der Produktion von anti-HIV-I-spezifischen Antikörpern durch den immunisierten Säuger; und

c.) Verschmelzen der Milzzellen des immunisierten Säugers mit einem Myelomzellstamm, wodurch eine Hybridzelle gebildet wird, welche anti-HIV-I-Antikörper sezerniert, wobei der Antikörper durch seine Spezifität gegen ein Epitop auf HIV-I-gp41 gekennzeichnet ist, welches von einer ersten Sequenz von Aminosäuren

```
Ile-His-Ser-Leu-Ile-Glu-Glu-Ser-Gln-Asn-Gln-
Gln-Glu-Lys-Asn-Glu-Gln-Glu-Leu-Leu-Glu-Leu-Asp-Lys
```

gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die flankierende Sequenz eine Aminosäuresequenz hat, die natürlichem HIV I gp41 entspricht, und wobei die flankierende Sequenz die erste Sequenz in die richtige antigenische Konformation versetzt.

**11.** Verfahren nach Anspruch 10, wobei die Hybridzelle einen Zellhybrid einer mit HIV I immunisierten Mäusemilzzelle umfaßt, die mit dem Myelomzellstamm SP2/0 verschmolzen ist.

**Patentansprüche für folgenden Vertragsstaat : ES**

**1.** Verfahren zur Herstellung eines monoklonalen Antikörpers, welches folgende Schritte umfaßt:

a.) Immunisieren eines Säugers mit HIV I-Antigen,

b.) Demonstrieren der Produktion von anti-HIV-I-spezifischen Antikörpern durch den immunisierten Säuger; und

c.) Verschmelzen der Milzzellen des immunisierten Säugers mit einem Myelomzellstamm, wodurch eine Hybridzelle gebildet wird, welche anti-HIV-I-Antikörper sezerniert, wobei der Antikörper durch seine Spezifität gegen ein Epitop auf HIV-I-gp41 gekennzeichnet ist, welches von einer ersten Sequenz von Aminosäuren

```
Ile-His-Ser-Leu-Ile-Glu-Glu-Ser-Gln-Asn-Gln-
Gln-Glu-Lys-Asn-Glu-Gln-Glu-Leu-Leu-Glu-Leu-Asp-Lys
```

gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die flankierende Sequenz eine Aminosäuresequenz hat, die natürlichem HIV I gp41 entspricht,und wobei die flankierende Sequenz die erste Sequenz in die richtige antigenische Konformation versetzt.

**2.** Verfahren nach Anspruch 1, wobei die Hybridzelle einen Zellhybrid einer mit HIV I immunisierten Mäusemilzzelle umfaßt, die mit dem Myelomzellstamm SP2/0 verschmolzen wurde.

**3.** Verfahren zum Nachweis einer Markierung in einer Probe, die den Kontakt mit HIV I anzeigt, folgendes umfassend:

- Bildung eines Antikörper/Antigen-Komplexes zwischen

dem Epitop auf HIV I gp41, welches von einer ersten Sequenz von Aminosäuren

```
            Ile-His-Ser-Leu-Ile-Glu-Glu-Ser-Gln-
Asn-Gln-Gln-Glu-Lys-Asn-Glu-Gln-Glu-Leu-Leu-Glu-Leu-Asp-Lys
```

gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die flankierende Sequenz eine Aminosäuresequenz hat, die natürlichem HIV I gp41 entspricht,
und einem gegen dieses Epitop spezifischen Antikörper;
- und den Nachweis der Anwesenheit oder Menge des gebildeten Antikörper/Antigen-Komplexes.

4. Verfahren nach Anspruch 3, wobei der Antikörper/Antigen-Komplex in einem immunometrischen, Konkurrenz-, Sandwich-, oder Agglutinations-Assayformat gebildet wird.

5. Verfahren nach Anspruch 4, wobei der Antikörper ein monoklonaler Antikörper ist.

6. Immunoassayverfahren zur Bestimmung der Anwesenheit oder der Menge von Antikörpern gegen HIV I gp41, die in der Testprobe vorhanden sein kann, folgendes umfassend:
- Inkubation der Testprobe mit
einem festphasenverankerten Bindungsmaterial, welches ein Zielepitop enthält, das die immunologischen Eigenschaften des Epitops auf HIV I gp41 hat, welches von einer ersten Sequenz von Aminosäuren

```
                Ile-His-Ser-Leu-Ile-
Glu-Glu-Ser-Gln-Asn-Gln-Gln-Glu-Lys-Asn-Glu-Gln-Glu-Leu-Leu-
Glu-Leu-Asp-Lys
```

gebildet wird, mit wenigstens einer flankierenden zweiten Aminosäuresequenz von mindestens 5 Aminosäuren Länge, entweder in 3'-Richtung zum Carboxyende oder in 5'-Richtung zum Aminoende von der ersten Sequenz, wobei die zweite flankierende Sequenz eine Aminosäuresequenz hat, die natürlichem HIV I gp41 entspricht, und mit einem Sondenantikörper, der spezifisch an das Zielepitop des Bindungsmaterials bindet;
- und Bestimmen der Anwesenheit oder Menge an Sondenantikörper, der an das Bindungsmaterial gebunden oder welcher ungebunden ist, als Anzeige für die Anwesenheit oder Menge des Antikörpers gegen HIV I gp41 in der Testprobe.

7. Immunoassayverfahren nach Anspruch 6, wobei das Bindungsmaterial teilweise gereinigtes HIV I, natürliches HIV I gp41-Antigen oder rekombinant-abgeleitetes gp 41 von voller Länge umfaßt.

**Revendications**
**Revendications pour les Etats contractants suivants : AT, BE, CH, DE, FR, GB, IT, LI, NL**

1. Anticorps monoclonal, caractérisé par sa spécificité pour un épitope se trouvant sur gp41 de HIV 1 formé, par une première séquence d'acides aminés

```
Ile–His–Ser–Leu–Ile–Glu–Glu–Ser–Gln–Asn–Gln–Gln–Glu–Lys–Asn–Glu–
Gln–Glu–Leu–Leu–Glu–Leu–Asp–Lys
```

et au moins une seconde séquence adjacente d'acides aminés ayant au moins 5 acides aminés de long, soit en 3' à l'extrémité terminale carboxyle soit en 5' à l'extrémité terminale amine de ladite première séquence, ladite séquence adjacente ayant une séquence d'acides aminés correspondant à la gp41 native de HIV 1, dans lequel ladite séquence adjacente amène ladite première séquence à une conformation antigénique appropriée.

**2.** Lignée cellulaire productrice d'anticorps d'hybridome, qui produit l'anticorps monoclonal selon la revendication 1.

**3.** Lignée cellulaire selon la revendication 2, laquelle comprend un hybride cellulaire d'une cellule splénique de souris immunisée par HIV 1, fusionnée à la lignée cellulaire de myélome SP2/0.

**4.** Procédé de détection d'un marqueur indicateur de l'exposition à HIV 1 dans un échantillon, comprenant la formation d'un complexe anticorps/antigène entre l'épitope se trouvant sur gp41 de HIV 1, formé par une première séquence d'acides aminés

$$\text{Ile–His–Ser–Leu–Ile–Glu–Glu–Ser–Gln–Asn–Gln–}$$

$$\text{Gln–Glu–Lys–Asn–Glu–Gln–Glu–Leu–Leu–Glu–Leu–Asp–Lys}$$

et au moins une seconde séquence adjacente d'acides aminés ayant au moins 5 acides aminés de long, soit en 3' à l'extrémité terminale carboxyle soit en 5' à l'extrémité terminale amine de ladite première séquence, ladite séquence adjacente ayant une séquence d'acides aminés correspondant à la gp41 native de HIV 1, et un anticorps spécifique de cet épitope ; et la détection de la présence ou de la quantité de complexe anticorps/antigène formé.

**5.** Procédé selon la revendication 4, dans lequel le complexe anticorps/antigène est formé dans un type de dosage immunométrique, compétitif, sandwich ou par agglutination.

**6.** Procédé selon la revendication 5, dans lequel l'anticorps est un anticorps monoclonal.

**7.** Procédé de dosage immunologique destiné à déterminer la présence ou la quantité d'un anticorps anti-gp41 de HIV 1 susceptible d'être présent dans un échantillon à tester, comprenant : l'incubation de l'échantillon à tester avec une substance de fixation fixée à une phase solide, contenant un épitope cible ayant les propriétés immunologiques de l'épitope se trouvant sur gp41 de HIV 1, formé par une première séquence d'acides aminés

$$\text{Ile–His–Ser–Leu–Ile–Glu–Glu–Ser–Gln–}$$

$$\text{Asn–Gln–Gln–Glu–Lys–Asn–Glu–Gln–Glu–Leu–Leu–Glu–Leu–Asp–Lys}$$

et au moins une seconde séquence adjacente d'acides aminés ayant au moins 5 acides aminés de long, soit en 3' à l'extrémité terminale carboxyle soit en 5' à l'extrémité terminale amine de ladite première séquence, ladite séquence adjacente ayant une séquence d'acides aminés correspondant à la gp41 native de HIV 1, et avec un anticorps sonde qui se fixe spécifiquement à l'épitope cible de la substance de fixation ; et la détermination de la présence ou de la quantité d'anticorps sonde fixée ou non fixée à la substance de fixation en tant qu'indication de la présence ou de la quantité d'anticorps anti-gp41 de HIV 1 dans l'échantillon à tester.

**8.** Procédé de dosage immunologique selon la revendication 7, dans lequel ladite substance de fixation comprend HIV 1 partiellement purifié, la gp41 native de HIV 1 ou la gp41 dérivée par recombinaison, de longueur totale.

**9.** Trousse diagnostique de détection de l'exposition à HIV 1, comprenant l'anticorps monoclonal selon la revendication 1 comme réactif.

**10.** Procédé de production d'un anticorps monoclonal selon la revendication 1, comprenant les étapes de :
a) immunisation d'un mammifère par l'antigène de HIV 1;
b) mise en évidence de la production d'anticorps spécifique anti-HIV 1 par ledit mammifère immunisé et
c) fusion des cellules spléniques dudit mammifère immunisé avec une lignée cellulaire de myélome, formant ainsi un hybridome qui sécrète l'anticorps anti-HIV 1, dans lequel ledit anticorps est

EP 0 335 134 B1

caractérisé par sa spécificité pour un épitope se trouvant sur gp41 de HIV 1, formé par une première séquence d'acides aminés

$$\text{Ile--His--Ser--Leu--Ile--Glu--Glu--Ser--Gln--Asn--Gln--Gln--Glu--Lys--Asn--}$$
$$\text{Glu--Gln--Glu--Leu--Leu--Glu--Leu--Asp--Lys}$$

et au moins une seconde séquence adjacente d'acides aminés ayant au moins 5 acides aminés de long, soit en 3' à l'extrémité terminale carboxyle soit en 5' à l'extrémité terminale amine de ladite première séquence, ladite séquence adjacente ayant une séquence d'acides aminés correspondant à la gp41 native de HIV 1, dans lequel ladite séquence adjacente amène ladite première séquence à une conformation antigénique appropriée.

**11.** Procédé selon la revendication 10, dans lequel ledit hybridome comprend un hybride cellulaire d'une cellule splénique de souris immunisée par HIV 1, fusionnée à la lignée cellulaire de myélome SP2/0.

**Revendications pour l'Etat contractant suivant : ES**

**1.** Procédé de production d'un anticorps monoclonal, comprenant les étapes de :
a) immunisation d'un mammifère par l'antigène de HIV 1 ;
b) mise en évidence de la production d'anticorps spécifique anti-HIV 1 par ledit mammifère immunisé et
c) fusion des cellules spléniques dudit mammifère immunisé avec une lignée cellulaire de myélome, formant ainsi un hybridome qui sécrète un anticorps anti-HIV 1, dans lequel ledit anticorps est caractérisé par sa spécificité pour un épitope se trouvant sur gp41 de HIV 1, formé par une première séquence d'acides aminés

$$\text{Ile--His--Ser--Leu--Ile--Glu--Glu--Ser--Gln--Asn--Gln--Gln--Glu--Lys--Asn--}$$
$$\text{Glu--Gln--Glu--Leu--Leu--Glu--Leu--Asp--Lys}$$

et au moins une seconde séquence adjacente d'acides aminés ayant au moins 5 acides aminés de long, soit en 3' à l'extrémité terminale carboxyle soit en 5' à l'extrémité terminale amine de ladite première séquence, ladite séquence adjacente ayant une séquence d'acides aminés correspondant à la gp41 native de HIV 1, dans lequel ladite séquence adjacente amène ladite première séquence à une conformation antigénique appropriée.

**2.** Procédé selon la revendication 1, dans lequel ledit hybridome comprend un hybride cellulaire d'une cellule splénique de souris immunisée par HIV 1, fusionnée avec la lignée cellulaire de myélome SP2/0.

**3.** Procédé de détection d'un marqueur indicateur de l'exposition à HIV 1 dans un échantillon, comprenant :
la formation d'un complexe anticorps/antigène entre l'épitope se trouvant sur gp41 de HIV 1, formé par une première séquence d'acides aminés

$$\text{Ile--His--Ser--Leu--Ile--Glu--Glu--Ser--Gln--Asn--Gln--Gln--Glu--Lys--Asn--Glu--}$$
$$\text{Gln--Glu--Leu--Leu--Glu--Leu--Asp--Lys}$$

et au moins une seconde séquence adjacente d'acides aminés ayant au moins 5 acides aminés de long, soit en 3' à l'extrémité terminale carboxyle, soit en 5' à l'extrémité terminale amine de ladite première séquence, ladite séquence adjacente ayant une séquence d'acides aminés correspondant à la gp41 native de HIV 1, et un anticorps spécifique de cet épitope ;
et
la détection de la présence ou de la quantité de complexe antigène/anticorps formé.

28

**4.** Procédé selon la revendication 3, dans lequel le complexe antigène/anticorps est formé dans un type de dosage immunométrique, compétitif, sandwich ou par agglutination.

**5.** Procédé selon la revendication 4, dans lequel l'anticorps est un anticorps monoclonal.

**6.** Procédé de dosage immunologique destiné à déterminer la présence ou la quantité d'un anticorps anti-gp41 de HIV 1, susceptible d'être présent dans un échantillon à tester, comprenant :

l'incubation de l'échantillon à tester avec une substance de fixation fixée à une phase solide, contenant un épitope cible ayant les propriétés immunologiques de l'épitope se trouvant sur gp41 de HIV 1, formé par une première séquence d'acides aminés

$$Ile-His-Ser-Leu-Ile-Glu-Glu-Ser-Gln-Asn-Gln-$$
$$Gln-Glu-Lys-Asn-Glu-Gln-Glu-Leu-Leu-Glu-Leu-Asp-Lys$$

et au moins une seconde séquence adjacente d'acides aminés ayant au moins 5 acides aminés de long, soit en 3' à l'extrémité terminale carboxyle soit en 5' à l'extrémité terminale amine de ladite première séquence, ladite séquence adjacente ayant une séquence d'acides aminés correspondant à la gp41 native de HIV 1, et avec un anticorps sonde qui se fixe spécifiquement à l'épitope cible de la substance de fixation ; et

la détermination de la présence ou de la quantité d'anticorps sonde fixé ou non fixé à la substance de fixation en tant qu'indication de la présence ou de la quantité d'anticorps anti-gp41 de HIV 1 dans l'échantillon à tester.

**7.** Procédé de dosage immunologique selon la revendication 6, dans lequel ladite substance de fixation comprend HIV 1 partiellement purifié, l'antigène gp41 native de HIV 1 ou la gp41 dérivée par recombinaison, de longueur totale.

FIGURE 1 B

FIGURE 1 A

FIGURE 2

FIGURE 3

Fig. 4

EP 0 335 134 B1

A  B  C  D

5-21-3
10-15-63
NM IgG
Mαrp41
NMS
RαP 58-130
NRS

FIGURE 5